# EUROPEAN PATENT SPECIFICATION

(11) **EP 2 720 744 B1**
(45) Date of publication and mention of the grant of the patent: **06.09.2017**
(21) Application number: 12803346.1
(22) Date of filing: 20.06.2012
(51) Int. Cl.: A61M 16/01, A61M 16/10, B01D 53/22, A61M 16/00, B01D 63/02, B01D 71/26, A61M 16/22, A61M 16/08

(54) **AN ANESTHETIC CIRCUIT**
NARKOTIKUMSKREISLAUF
CIRCUIT ANESTHÉSIQUE

(30) Priority: 20.06.2011 US 201161499038 P
(43) Date of publication of application: 23.04.2014
(73) Proprietor: DMF Medical Incorporated, Halifax, NS B3H 0A8 (CA)
(72) Inventor: SCHMIDT, Klaus Michael, Halifax, NS B3H 4S9 (CA)
(74) Representative: Gordon, Jennifer Claire
(86) International application number: PCT/CA2012/000605
(87) International publication number: WO 2012/174649

(56) References cited:
- EP-A1- 1 057 521
- EP-A1- 1 424 092
- EP-A1- 1 803 479
- WO-A1-99/10034
- WO-A1-2004/050154
- WO-A1-2008/142665
- WO-A1-2008/142665
- US-A- 3 489 144
- US-A1- 2005 145 107
- US-A1- 2007 017 516
- US-A1- 2007 286 783
- US-A1- 2009 145 761
- US-A1- 2010 258 117
- US-A1- 2011 041 848
- LAGORSSE ET AL: "Xenon recycling in an anaesthetic closed-system using carbon molecular sieve membranes", JOURNAL OF MEMBRANE SCIENCE, ELSEVIER, vol. 301, no. 1-2, 2 August 2007 (2007-08-02), pages 29-38, XP022182905, ISSN: 0376-7388, DOI: 10.1016/J.MEMSCI.2007.05.032

## Description

### FIELD

This invention relates to an anesthetic circuit to anesthetize a patient. Also described is a method of using an anesthetic circuit to anesthetize a patient.

### INTRODUCTION

Anesthetic agents are commonly used to anesthetize a patient during a medical procedure. To keep the stress level low and relax the patient, the patient has to be asleep for many medical procedures. Anesthetic circuit systems wherein anesthetic agent is partially re-used after being delivered to the patient are known in the art. The benefit is that less anesthetic agent is used. This is financially beneficial due to the relatively high cost of most anesthetic agents. The use of less anesthetic agents may also be good for the environment since some anesthetic agents, such as the halogenated hydrocarbon sevoflurane, for example, are greenhouse fluids.

Carbon dioxide is formed in the cell and is released though the alveoli of the lungs during expiration at a level of around 5% of the expiratory fluid mixture. The concentration at the end of expiration is called the end tidal carbon dioxide value. The inspiratory level of carbon dioxide is normally well below 0.5%. Having excessive levels of carbon dioxide in the blood of the patient will decrease the pH value of the blood (acidosis) and will, if not treated properly, affect the patient's brain activity and may eventually lead to unconsciousness and death.

When the patient inhales the anesthetic agent in a fluid mixture, the anesthetic agent passes through the alveoli of the lungs into the patient's blood. The patient exhales a fluid mixture comprising, among other components, exhaled anesthetic, exhaled oxygen and exhaled carbon dioxide. Due to the operation of the human's lungs, the carbon dioxide content of the exhaled fluid mixture is higher than that of the inhaled fluid mixture. Furthermore, the oxygen content of the exhaled fluid mixture is lower than that of the inhaled fluid mixture. To be able to re-use the fluid mixture (containing the exhaled anesthetic fluid), the carbon dioxide of the exhaled fluid mixture must be lowered to levels suitable for re-inhalation.

Anesthetic circuits aimed at decreasing the amount of carbon dioxide fluid re-inhaled by the patient are known in the art. Some in the industry have focused on decreasing the carbon dioxide content in the exhaled mixture, along with trying to preserve exhaled oxygen and exhaled molecular anesthetic agent within the anesthetic circuit for re-inhalation. Their desire to preserve exhaled oxygen fluid is premised on the notion that oxygen needs to be provided as part of the inhaled mixture in an appropriate level to keep the oxygen saturation in the patient's blood high enough to allow for proper metabolism. Many publications focus on separating or binding the CO₂ specifically and therefore separate it from the fluid mixture containing the anesthetic agent.

Some conventional anesthetic circuits use carbon dioxide absorbers to reduce exhaled carbon dioxide within the anesthetic circuit. In some cases, soda lime or baralyme, for example, are used. Sevoflurane and other anesthetic vapors can react with these carbon dioxide absorbers to produce harmful chemicals such as compound A. Compound A has been found to have negative effects such as nephro and cerebo toxic effects.

In other conventional systems, a membrane impregnated with a substance that is chemically reactive with carbon dioxide (and, in some cases, anesthetic agent) is used to reduce the amount of exhaled carbon dioxide from an anesthetic circuit. For example, membranes comprising amino acids or amine groups that are chemically reactive with carbon dioxide are known in the art. The reactive sites may degrade or become contaminated overtime, which requires the membrane to be disposed of and replaced.

Specific examples of selective membranes known in the art that separate an anesthetic from at least one other fluid include: United States Patent No. 2007/0017516 to Schmidt, United States Patent Application No. 2010/0031961 to Schmidt, and United States Patent No. 2009/0126733 to Kulkarni et al. A selective, porous membrane is described in The Journal of Membrane Science Article "Xenon recycling in an anaesthetic closed-system using carbon molecular sieve membranes" (S. Lagorsse, F.D. Magalhães, A. Mendes; Journal of Membrane Science 301 (2007) 29-38). An anesthetic circuit incorporating a porous membrane is described in Figure 1 of this reference.

There exists a need for an improved anesthetic circuit in which exhaled molecular anesthetic agent can be effectively retained and re-circulated to the patient.

### SUMMARY

The following summary is provided to introduce the reader to the more detailed discussion to follow. The summary is not intended to limit or define the claims.

According to the invention, an anesthetic circuit for treating a patient is provided comprising:
a flow passage (12);
a molecular anesthetic agent inlet (14) in fluid communication with the flow passage for introducing an external anesthetic agent comprising a volatile molecular anesthetic agent (16) into the flow passage;
an exit outlet (22) in fluid communication with the flow passage for providing at least the external anesthetic agent comprising a volatile molecular anesthetic agent to the patient;
an entry inlet (36) for receiving an exhaled fluid mixture (26) from the patient, the exhaled fluid mixture comprising an exhaled oxygen (28), an exhaled carbon dioxide (30) and an exhaled volatile molecular anesthetic agent (34), the flow passage being in fluid communication with the entry inlet for receiving the exhaled fluid mixture from the entry inlet,
a membrane (38) comprising at least one polymeric material, in fluid communication with the flow passage, located downstream from the entry inlet, and at least partially impervious to the exhaled volatile molecular anesthetic agent to at least partially retain the exhaled volatile molecular anesthetic agent in the flow passage after the exhaled fluid mixture contacts the membrane, wherein the membrane comprises a dense membrane and has a non-porous consistency,
the membrane is pervious to the exhaled oxygen such that the membrane has an exhaled oxygen-to-exhaled volatile molecular anesthetic agent selectivity of greater than 1,
the membrane is pervious to the exhaled carbon dioxide such that the membrane has an exhaled carbon dioxide-to-exhaled volatile molecular anesthetic agent selectivity of greater than 1,
the membrane is capable, upon contact with the exhaled fluid mixture, of leaving a modified fluid mixture (42) in the flow passage having a lower amount of the exhaled carbon dioxide than the exhaled fluid mixture, and
the exit outlet is located downstream from the membrane and provides at least the modified fluid mixture to the patient (20); and
the membrane is inert with respect to the exhaled volatile molecular anesthetic agent and inert with respect to the exhaled carbon dioxide; and
a fluid inlet (50) for introducing an external fluid into the flow passage to be added to the modified fluid mixture provided to the patient.

The membrane may have an exhaled oxygen-to-exhaled molecular anesthetic agent selectivity of at least 2. Optionally, the membrane has an exhaled oxygen-to-exhaled molecular anesthetic agent selectivity of at least 3, 4, 5, 10, 50, 100 or 250.

The membrane may have an exhaled carbon dioxide- to-exhaled molecular anesthetic agent selectivity of at least 2. Optionally, the membrane has an exhaled carbon dioxide-to-exhaled molecular anesthetic agent selectivity of at least 3, 4, 5, 10, 50, 100 or 250.

The membrane may be entirely made up of polymeric material.

In some embodiments, the membrane is configured such that a secondary oxygen located external to the flow passage passes through the membrane and into the flow passage.

In some embodiments, the anesthetic circuit further comprises an external oxygen source for enriching the external fluid with external oxygen.

In some embodiments, the anesthetic circuit comprises one flow generator for facilitating flow of the exhaled fluid mixture and the modified fluid mixture through the flow passage.

In some embodiments, the anesthetic circuit comprises a turbulence-inducing component in the flow passage to create a turbulent flow of the exhaled fluid mixture at the membrane to increase contact between the exhaled fluid mixture and the membrane.

The exhaled molecular anesthetic agent is a volatile anesthetic agent and the membrane is at least partially impervious to the volatile anesthetic agent.

The exhaled molecular anesthetic agent may comprise a polyhalogenated ether.

The exhaled molecular anesthetic agent may include at least one of sevoflurane, isoflurane or desflurane.

The exhaled molecular anesthetic agent may have a molecular weight of greater than 168 g/mol.

In some cases, a carbon dioxide absorbing material is located on a side of the membrane that is external to the flow passage, wherein the membrane separates the carbon dioxide absorbing material from the exhaled molecular anesthetic agent retained in the flow passage to impede the exhaled molecular anesthetic agent from contacting the carbon dioxide absorbing material. The carbon dioxide absorbing material may comprise at least one of: soda lime, alkanolime, alkanolamine, amino compounds, alkali salts of amino acids, glycine, DL-alanine, beta-alanine, serine, threonine, isoleucine, DL-valine, piperazine-2-carboxilic acid, proline, arginine, gamma-aminobutyric acid, ornithine, potassium glycinate, potassium threonate, taurine, creatine and histidine.

In some embodiments, the exhaled fluid mixture comprises a metabolic product including acetaldehyde, acetone, ethane, ethylene, hydrogen, isoprene, methane, methylamine or pentane. In this case, the membrane may be pervious to the metabolic product. In this case, the exhaled fluid mixture contacts the membrane to leave a modified fluid mixture in the flow passage having a lower amount of the metabolic product than the exhaled fluid mixture.

The membrane may be a polyhalocarbon membrane. More specifically, the membrane may be a polymethylpentene membrane. The membrane may be a polysiloxane membrane. More specifically, the membrane may be a polydimethyl siloxane membrane.

The membrane is a dense membrane. The membrane may be a dense polymethylpentene membrane.

The membrane may be an asymmetric membrane comprising hollow fibers having at least one wall comprising a porous support layer and a dense layer.

In some cases, the membrane comprises a glassy polymer, a polymeric size selective membrane or a composite polymer membrane.

The membrane may be free of any amino acids.

Also described is a method for anesthetic treatment of a patient. The method comprises:
introducing an external anesthetic agent comprising a volatile molecular anesthetic agent towards and into the patient via a flow passage;
directing an exhaled fluid mixture comprising an exhaled oxygen, an exhaled carbon dioxide and an exhaled volatile molecular anesthetic agent away from and out of the patient into the flow passage;
advancing the exhaled fluid mixture through the flow passage towards and into contact with a membrane comprising polymeric material and in fluid communication with the flow passage, wherein the membrane comprises a dense membrane and has a non-porous consistency, and wherein the membrane is inert with respect to the exhaled volatile molecular anesthetic agent and inert with respect to the exhaled carbon dioxide;
transferring more of the exhaled carbon dioxide than the exhaled volatile molecular anesthetic agent from the exhaled fluid mixture through the membrane and out of the flow passage after the exhaled fluid mixture contacts the membrane to leave a modified fluid mixture in the flow passage, wherein the modified fluid mixture has a lower concentration of the exhaled carbon dioxide than the exhaled fluid mixture;
transferring exhaled oxygen through the membrane after the exhaled fluid mixture contacts the membrane to leave a modified fluid mixture in the flow passage, wherein the membrane has an exhaled oxygen-to-exhaled molecular anesthetic agent selectivity of greater than 1; and
advancing the modified fluid mixture through the flow passage toward the patient to provide at least the modified fluid mixture to the patient.

### DRAWINGS

Reference is made in the description of various embodiments to the accompanying drawings, in which:
Figure 1 is a side view of an exemplary anesthetic circuit in accordance with an embodiment of the invention;
Figure 2 is a side view of the anesthetic circuit of Figure 1 further comprising an external oxygen source;
Figure 3a is a side view of an anesthetic circuit in accordance with an alternative embodiment of the invention having a compressible member;
Figure 3b is a side view of an anesthetic circuit in accordance with an alternative embodiment of the invention having a membrane located between a flow generator and the remainder of a flow passage;
Figure 3c is a side view of an anesthetic circuit in accordance with an alternative embodiment of the invention having a bellow;
Figure 4 is a side view of the anesthetic circuit of Figure 1 further comprising a turbulence-inducing member;
Figure 5 is a side view of the anesthetic circuit of Figure 1 further comprising a plurality of membranes;
Figure 6a is a side of view of the anesthetic circuit illustrating fluid flows relative to the membrane;
Figure 6b is a side of view of the anesthetic circuit illustrating fluid flows relative to the membrane;
Figure 7 is a side view of the anesthetic circuit of Figure 1 further comprising a carbon dioxide absorbing material;
Figure 8 is a side view of a membrane configuration in accordance with an embodiment of the invention;
Figure 9 is a side view of a membrane configuration in accordance with an alternative embodiment of the invention;
Figure 10 is a side view of a membrane configuration in accordance with another embodiment of the invention;
Figure 11 is a side view of a membrane configuration in accordance with yet another embodiment of the invention;
Figure 12 is a top microscopic view of a membrane configuration in accordance with yet another embodiment of the invention;
Figure 13 provides a perspective view of the QUADROX-D^{™} oxygenator;
Figure 14 provides a side view of an example oxygenator;
Figure 15 provides a side view of the oxygenator of Figure 14, rotated by 90° relative to the flow passage;
Figure 16 provides a microscopic view of a dense layer of an OXYPLUS^{™} membrane;
Figure 17 provides a microscopic view of a porous support layer of an OXYPLUS^{™} membrane;
Figure 18 provides a partial plan view of a hollow fiber of an OXYPLUS^{™} membrane;
Figure 19 provides a microscopic plan view of the dense layer and porous support layer of an OXYPLUS^{™} membrane;
Figure 20 provides a schematic representation of the ACCUREL^{™} production process;
Figure 21 provides a microscopic plan view of a hollow fiber of an ULTRAPHOBIC^{™} membrane;
Figure 22 provides a microscopic view of a dense layer of an **UL**TRAPHOBIC^{™} membrane
Figure 23 provides a microscopic view of a porous support layer of an ULTRAPHOBIC^{™} membrane; and
Figure 24 provides a microscopic plan view of a dense layer and a porous support layer of an ULTRAPHOBIC^{™} membrane.

### DESCRIPTION OF VARIOUS EMBODIMENTS

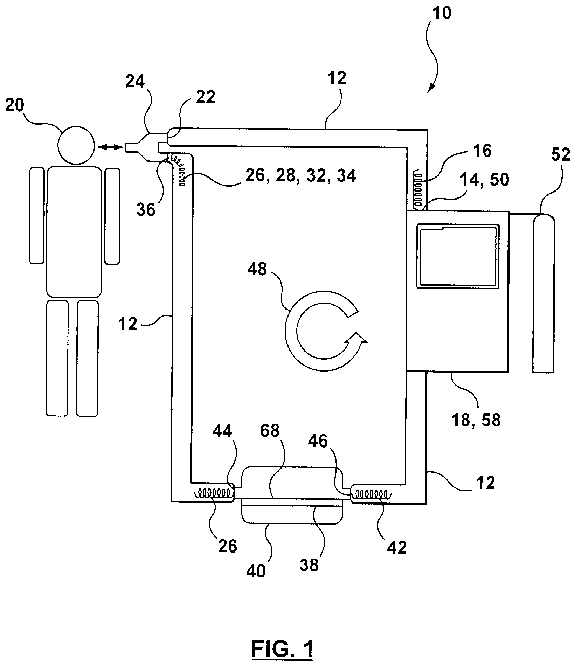

Figure 1 illustrates an exemplary anesthetic circuit 10 for treating a patient. As illustrated in Figure 1, anesthetic circuit 10 comprises a flow passage 12. Flow passage 12 provides a passageway for transferring fluid to and from patient 20. It should be noted that fluid, as discussed herein, includes a gas or combination of gases, or a liquid or combination of liquids. Liquids may be present in vapor form, for example. The term fluid may also encompass a mixture of fluids and liquids (which, in some cases, may be in vapor form). In some cases, flow passage 12 provides a hollow conduit. Flow passage 12 may be flexible tubing, for example. Flow passage 12 may be made of a polymeric material, such as plastic. In some cases, anesthetic circuit 10 may be a ventilation system.

An anesthetic inlet 14 is in fluid communication with flow passage 12. Anesthetic inlet 14 introduces at least the external anesthetic agent 16 into flow passage 12. Figure 1 illustrates anesthetic inlet 14 of flow passage 12 in fluid communication with an anesthetic machine 18, as is conventionally used to deliver external anesthetic agent 16 to patient 20. External anesthetic agent 16 may be stored within and delivered to flow passage 12 by anesthetic machine 18. Anesthetic machine 18 may monitor the flow rates of the fluids travelling through flow passage 12. Anesthetic machine 18 may also be used to monitor the physical characteristics and vital signs of patient 20. Patient 20 is illustrated in Figure 1 as being a human being; however, patient 20 may be any human, animal, cell or organism. Anesthetic circuit 10 may be used to treat any living cells or organisms, such as for example humans and animals. Anesthetic circuit 10 may be used to treat domestic pets, such as dogs and cats, for example.

An exit outlet 22 is also in fluid communication with flow passage 12. Exit outlet 22 provides at least external anesthetic agent 16 to patient 20. External anesthetic agent 16 will initially anesthetize patient 20, when the anesthetic process commences by delivery of external anesthetic agent 16 to the
airway of patient 20, via exit outlet 22. Exit outlet 22 may be configured to be directly received by the airway of patient 20, for delivery of fluid from flow passage 12 to patient 20. Alternatively, exit outlet 22 may engage a Y-piece 24 that is received by the airway of patient 20. Patient 20 breathes in the external anesthetic agent 16 through his/her airway, thereby delivering the anesthetic agent to the patient's lungs.

An exchange occurs in the alveoli of the lungs of patient 20 such that patient 20 breathes out transformed exhaled fluid mixture 26. Exhaled fluid mixture 26 comprises exhaled oxygen 28, exhaled carbon dioxide 30 and exhaled molecular anesthetic agent 34.

Exhaled molecular anesthetic agent 34 is a molecular anesthetic agent, which may or may not be mixed with other fluids in addition to exhaled oxygen 28 and exhaled carbon dioxide 30. Those skilled in the art will appreciate that molecular anesthetic agents have more than one different atomic element bonded together to form a molecule. For example, sevoflurane is a molecular anesthetic agent that has the chemical form (1,1,1,3,3,3-hexafluoro-2-(fluoromethoxy)propane). In turn, sevoflurane comprises different elements fluorine, carbon and oxygen bonded together. By contrast, noble gases consist of only one atomic element that is not bonded to other atomic elements. For example, Xenon anesthetic is made up of only xenon atoms, and argon is made up only argon atoms. Exhaled molecular anesthetic agent 34 may originate from external anesthetic agent 16, which, in this case, comprises a molecular anesthetic agent. In some cases, exhaled molecular anesthetic agent 34 is a molecular anesthetic agent that was solved in the patient's body (i.e. after cardiac surgery). In some cases, exhaled molecular anesthetic agent 34 comprises a molecular anesthetic agent that was partially solved in the patient's body, and partially contained in external anesthetic agent 34 that was introduced to the patient's airway. In some embodiments, exhaled molecular anesthetic agent 34 is the only exhaled anesthetic agent. In some embodiments, exhaled molecular anesthetic agent 34 is mixed with other non-molecular anesthetic agents.

Optionally, exhaled molecular anesthetic agent 34 comprises a polyhalogenated ether. Exhaled molecular anesthetic agent 34 may be hydrophobic (i.e. in gaseous form it dissolves in oil better than water, and in liquid form it is freely miscible with water). Non-limiting examples of exhaled molecular anesthetic agent 34 include: sevoflurane, desflurane or isoflurane. Molecular anesthetic agent 34 may be entirely comprised of one of sevoflurane, desflurane or isoflurane, or a mixture thereof.

Exhaled molecular anesthetic agent 34 is a volatile anesthetic. Volatile anesthetics are liquid at room temperature (optionally 20°C at 1 atm), but readily evaporate under reduced pressure. Optionally, exhaled molecular anesthetic agent 34 has a vapor pressure at 20°C of between approximately 155 mmHg and 670 mmHg. Optionally, exhaled molecular anesthetic agent 34 has a vapor pressure at 20°C of between approximately 250 mmHg and 500mmHg.

Optionally, exhaled molecular anesthetic agent 34 has a boiling point at 760mm in the range of approximately 20°C to 60°C.

Optionally, exhaled molecular anesthetic agent 34 has a molecular weight of at least 150 g/mol. Optionally, exhaled molecular anesthetic agent has a molecular weight of at least 168 g/mol. Notably, by contrast, Xenon (which is an atomic anesthetic) has a lesser molecular weight of approximately 131.3 g/mol.

Anesthetic circuit 10 has an entry inlet 36 for receiving exhaled fluid mixture 26 from patient 20. Flow passage 12 is in fluid communication with entry inlet 36 for receiving exhaled fluid mixture 26 from entry inlet 36. Entry inlet 36 may be configured to be directly received by the airway of patient 20, for delivery of fluid from patient 20 to flow passage 12. Entry inlet 36 may be a one-way valve. Alternatively, entry inlet 36 may engage a Y-piece 24 that is received by
the airway of patient 20. Entry inlet 36 may be separate and distinct from exit outlet 22, as exemplified in Figure 1. Alternatively, entry inlet 36 and exit outlet 22 may be one aperture formed within flow passage 12, for delivering fluid to and from patient 20. In some embodiments, anesthetic inlet 14 is separate and distinct from entry inlet 36 and exit outlet 22. Exit outlet 22 may be a one-way valve. In some embodiments, at least one of entry inlet 36 and exit outlet 22 may function as anesthetic inlet 14. Anesthetic inlet 14 may be an injector for liquid anesthetic agents.

As exemplified in Figure 1, anesthetic circuit 10 comprises a membrane 38 comprising at least one polymeric material and in fluid communication with flow passage 12. As exemplified in Figure 1, membrane 38 may be contained within a membrane housing 40. Alternatively, membrane 38 may fit into an aperture in a wall of flow passage 12, in the absence of membrane housing 40. When membrane 38 fits into an aperture in a wall of flow passage 12, membrane 38 may be fixedly attached to the remainder of a wall of flow passage 12, or formed integrally therewith. In some cases, membrane 38 spans internally between the walls of flow passage 12.

As exemplified in Figure 1, membrane 38 is located downstream from entry inlet 36. In this embodiment, when the exhaled fluid mixture 26 travels through flow passage 12 and after it contacts the membrane 38, a portion of exhaled fluid mixture 26 passes through membrane 38 and out of flow passage 12, to leave a modified fluid mixture 42 in flow passage 12.

When membrane 38 is contained in membrane housing 40, exhaled fluid mixture 26 may be received into membrane housing 40 through housing inlet 44. After exhaled fluid mixture 26 contacts the membrane 38 within membrane housing 40, a modified fluid mixture 42 is created within membrane housing 40. Modified fluid mixture 42 may exit the membrane housing 40 via housing outlet 46. Once the modified fluid mixture 42 exits the membrane housing 40, it may carry on through flow passage 12.

Membrane 38 comprises at least one polymeric material. In some embodiments, membrane 38 is entirely made up of polymeric material. In some embodiments, membrane 38 is entirely made up of only one polymeric material. In some embodiments, membrane 38 comprises a polysiloxane and is thereby a polysiloxane membrane. More specifically, membrane 38 may comprise polydimethyl siloxane and thereby be a polydimethyl membrane. In some embodiments, membrane 38 comprises a halocarbon polymer and is thereby a polyhalocarbon membrane. More specifically, membrane 38 may comprise polymethylpentene and thereby be a polymethylpentene membrane.

Exit outlet 22 is located downstream from membrane 38 and provides at least the modified fluid mixture 42 to patient 20. Entry inlet 36 may be located upstream from membrane 38.

As shown in Figure 1, anesthetic circuit 10 comprises a fluid inlet 50 for introducing external fluid from external fluid source 52 to be added to modified fluid mixture 42 in flow passage 12. Fluid inlet 50 may be an independent inlet in fluid communication with flow passage 12. Alternatively, anesthetic inlet 14 may also serve as fluid inlet 50, as illustrated in Figure 1. Fluid inlet 50 may allow additional fresh fluid (ex. oxygen or air) to be added to flow passage 12 and provided to patient 20 if needed, if the oxygen level within flow passage 12 falls below acceptable an level to support patient 20. Oxygen replenishment may be required if, for example, patient 20 increases his/her metabolic rate. Oxygen replenishment may also be required if significant amounts of oxygen exit the flow passage 12 via membrane 38. It should be noted that it is generally cheaper (per unit volume) to add air or oxygen to flow passage 12 than to add external anesthetic agent 16 to flow passage 12. External air source 52 may be a tank containing compressed, pressurized air therein.

In the manner outlined above, fluids may at least partially recirculate through flow passage 12. An example fluid flow direction 48 is illustrated in Figure 1.

In an alternative embodiment to that illustrated in Figure 1, membrane 38 may be located in a portion of flow passage 12 (not shown) that is external to the portion of flow passage 12 that moves fluid in a circular loop corresponding to fluid flow direction 48 (not shown). In some cases, membrane 38 may be located in a branch passage of flow passage 12 (not shown) located between fluid source 52 and the portion of flow passage 12 that moves fluid in a circular loop corresponding to fluid flow direction 48. In some embodiments, membrane 38 may be located in anesthetic machine 18.

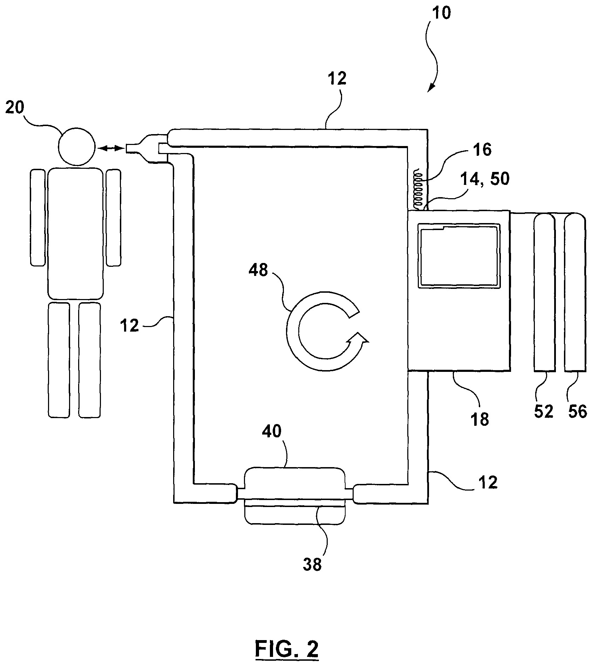

As shown in Figure 2, in some cases, anesthetic circuit 10 comprises an external oxygen source 56 for enriching the external fluid with external oxygen.. External oxygen source 56 may be a tank containing compressed, pressurized oxygen fluid therein. The external oxygen may be delivered through fluid inlet 50.

Returning to Figure 1, anesthetic circuit 10 may comprise at least one flow generator 58 for facilitating flow of exhaled fluid mixture 26 and modified fluid mixture 42 through flow passage 12. In the embodiment shown in Figure 1, anesthetic machine 18 may serve as flow generator 58, as is commonly known in the art. In some cases, a plurality of flow generators 58 may be provided. As an example, a first flow generator may drive the flow of the exhaled fluid mixture 26 and a second flow generator may drive the flow of modified fluid mixture 42. Examples of flow generator 58 include a motor, fan, pump or vacuum capable of advancing fluids through flow passage 12.

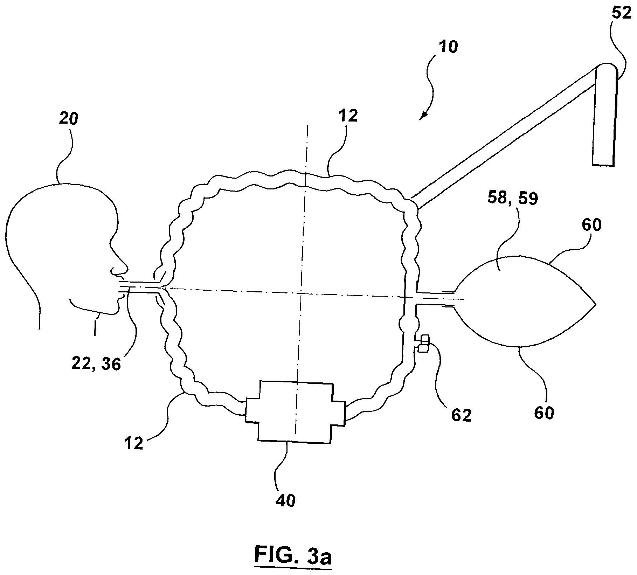

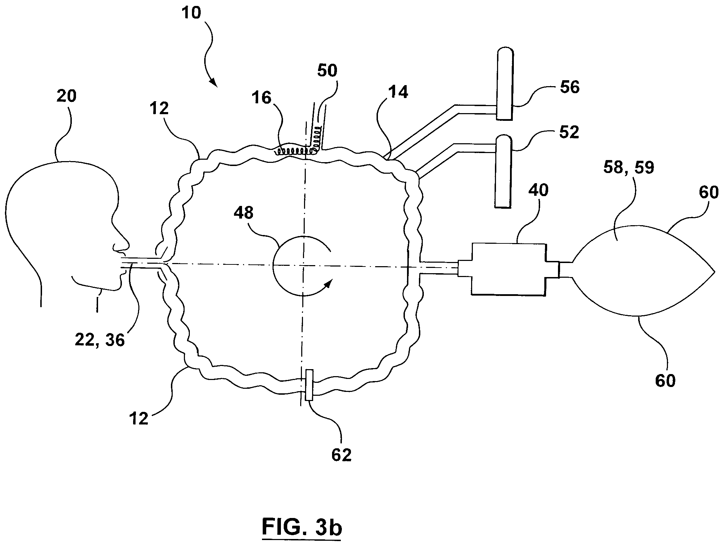

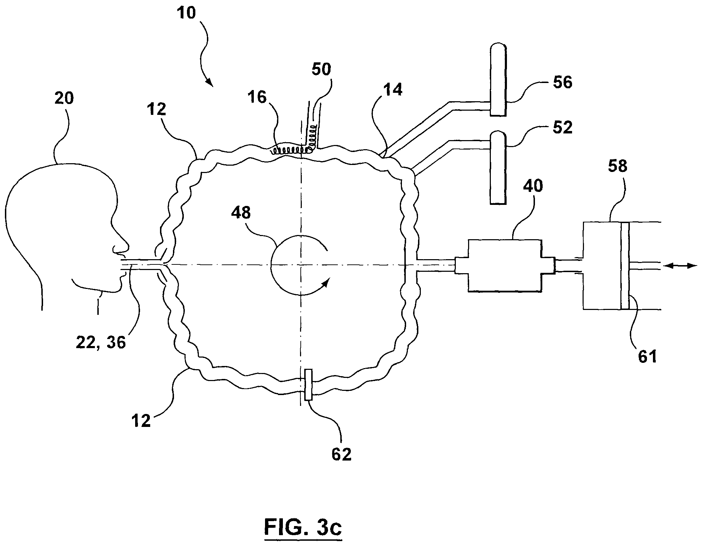

In an alternative embodiment illustrated in Figure 3a, flow generator 58 comprises compressible member 59. As illustrated in Figure 3, compressible member 59 comprises opposing walls 60. When opposing walls 60 are moved towards one another, a positive driving pressure is created in flow passage 12. Opposing walls 60 may be flexible and resiliently biased such that after they are compressed and released, they return to their uncompressed state. This motion creates a negative driving pressure in flow passage 12. Opposing walls 60 may be manually compressible by a human hand.

In some cases, as illustrated in Figure 3a, flow passage 12 comprises at least one valve 62 for releasing fluid within flow passage 12, if necessary.

As exemplified in Figure 3b, membrane housing 40 (having a membrane therein, not shown) may also be located between the remainder of flow passage 12 and flow generator 58. As exemplified in Figure 3b, flow generator 58 may comprise a compressible chamber 59 having opposing walls 60.

In an alternative embodiment shown in Figure 3c, flow generator 58 comprises a bellow comprising a plunger 61 for generating fluid flow.

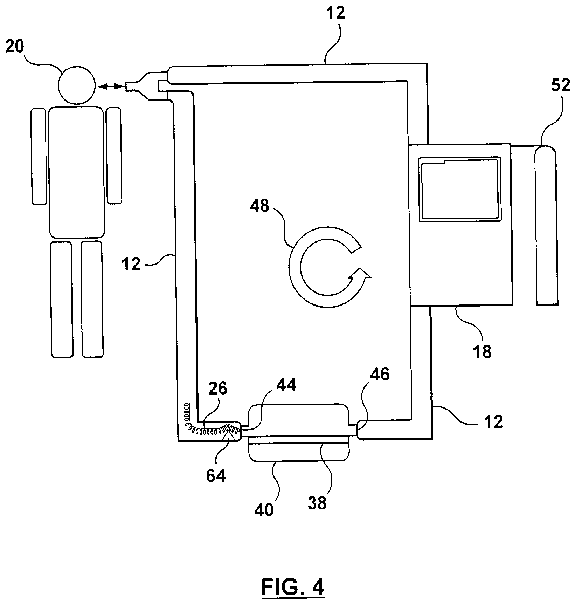

As shown in Figure 4, anesthetic circuit 10 may also comprise a turbulence-inducing component 64. The turbulence-inducing component 64 creates a turbulent flow of exhaled fluid mixture 26 at membrane 38 to increase contact between exhaled fluid mixture 26 and membrane 38. Turbulence-inducing component 64 may be any object placed within flow passage 12, as illustrated in Figure 4, such that the fluids travelling therethrough are forced to flow around the object. Alternatively, turbulence-inducing component 64 may comprise a change in geometry within at least one wall of flow passage 12. Optionally, the change in geometry is abrupt, so as to generate fluid flow eddies within flow passage 12. The turbulence-inducing component 64 is optionally located upstream and adjacent to the membrane 38. When membrane 38 is contained in membrane housing 40, turbulence-inducing component 64 may be located upstream and adjacent to the housing inlet 44, as illustrated in Figure 4. Turbulence-inducing component 64 may also be located within membrane housing 40.

As exemplified in Figures 3a-3c, flow passage 12 may have a nonuniform cross-section throughout its length so as to promote turbulent fluid flow through flow passage 12.

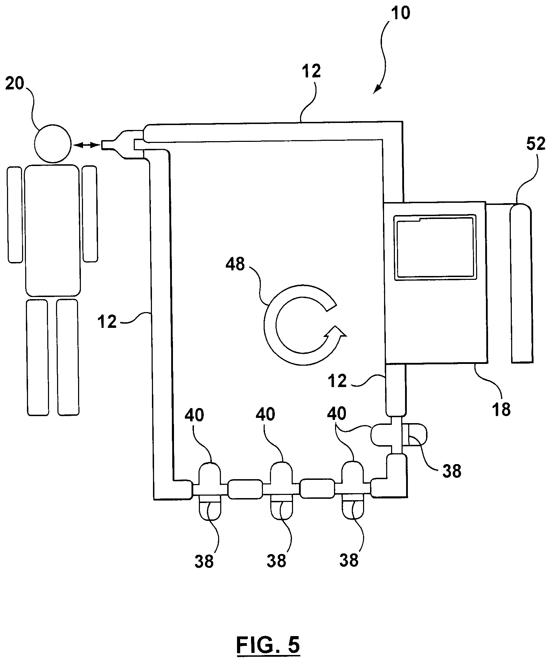

As exemplified in Figure 5, anesthetic circuit 10 may comprise a plurality of membranes 38. Each membrane 38 may be contained within its own membrane housing 40, as exemplified in Figure 5.

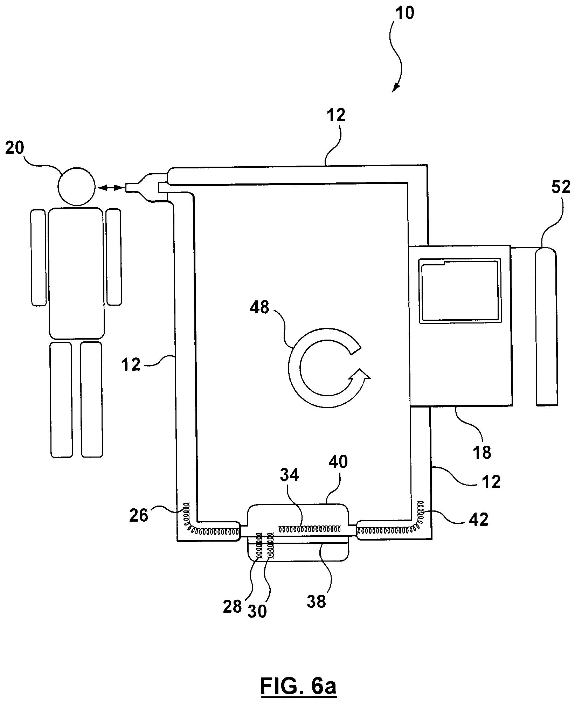

As exemplified in Figure 6a, membrane 38 is at least partially impervious to the exhaled molecular anesthetic agent 34 to at least partially retain exhaled molecular anesthetic agent 34 in flow passage 12 after the exhaled fluid mixture 26 contacts the membrane 38. In some cases, membrane 38 is substantially impervious to the exhaled molecular anesthetic agent 34 to substantially retain exhaled molecular anesthetic agent 34 in flow passage 12 after the exhaled fluid mixture 26 contacts the membrane 38. Optionally, membrane 38 is substantially impervious to exhaled molecular anesthetic agent 34. In some cases, membrane 38 is configured to permeate less than 5% of the molecular anesthetic agent. Optionally, membrane 38 is substantially pervious to atomic anesthetic agents (i.e. noble gases, including xenon, for example).

Membrane 38 is pervious to exhaled oxygen 28 such that membrane 38 has an exhaled oxygen-to-exhaled molecular anesthetic agent selectivity of greater than 1. In other words, more exhaled oxygen 28 leaves flow passage 12 through membrane 38 than exhaled molecular anesthetic agent 34. Membrane 38 may be pervious to exhaled oxygen 28 such that membrane 38 has an exhaled oxygen-to-exhaled molecular anesthetic agent selectivity of at least two 2. In other words, at least twice as much exhaled oxygen 28 may leave flow passage 12 through membrane 38 than exhaled molecular anesthetic agent 34. Optionally, membrane 38 may be pervious to exhaled oxygen 28 such that is has an exhaled oxygen-to-exhaled molecular anesthetic agent selectivity of at least 3, 4, 5, 10, 50, 100 or 250. Optionally, membrane 38 is substantially pervious to exhaled oxygen.

Membrane 38 is pervious to exhaled carbon dioxide such that membrane 38 has an exhaled carbon dioxide-to-exhaled molecular anesthetic agent selectivity of greater than 1. In other words, more exhaled carbon dioxide 30 leaves flow passage 12 through membrane 38 than exhaled molecular anesthetic agent 34. Membrane 38 may be substantially pervious to exhaled carbon dioxide such that membrane 38 has an exhaled carbon dioxide-to-exhaled molecular anesthetic agent selectivity of at least 2. In other words, at least twice as much exhaled carbon dioxide 30 may leave flow passage 12 through membrane 38 than exhaled molecular anesthetic agent 34. Optionally, membrane 38 may be pervious to exhaled carbon dioxide 30 such that is has an exhaled carbon dioxide-to-exhaled molecular of at least 3, 4, 5, 10, 50, 100 or 250. Optionally, membrane 38 is substantially pervious to exhaled carbon dioxide.

Exhaled fluid mixture 26 contacts membrane 38 to leave modified fluid mixture 42 in flow passage 12. The modified fluid mixture 42 has a lower amount of exhaled carbon dioxide 30 than exhaled fluid mixture 26. In other words, the amount of exhaled carbon dioxide 30 in modified fluid mixture 42 is less than the amount of exhaled carbon dioxide 30 in exhaled fluid mixture 26. In some cases, modified fluid mixture 42 has a lower amount of exhaled oxygen 28 than exhaled fluid mixture 26. Figure 6a shows exhaled oxygen 28 and exhaled carbon dioxide 30 passing through membrane 38 and out of flow passage 12 after exhaled fluid mixture 26 contacts membrane 38.

Many conventional membranes used in anesthetic circuits focus on retaining exhaled oxygen 28 in flow passage 12. It is advantageous, in certain cases, to let some of exhaled oxygen 28 to pass through membrane 38. Figure 6a showns exhaled oxygen 28 passing out of flow passage 12 through membrane 38. In some embodiments, a substantial amount of exhaled oxygen 28 is permitted to leave the system. External oxygen can be relatively cheaply replenished into flow passage 12 to account for the exhaled oxygen 28 lost through membrane. The cost of external oxygen is substantially less than the cost of external anesthetic agent 16. It is advantageous to at least partially (optionally, substantially) retain exhaled molecular anesthetic agent 34, while allowing some (optionally, a substantial amount of) exhaled oxygen 28 to pass through membrane 38 and out of anesthetic circuit 10. Membranes that have these properties provide some advantages over conventional membranes that have a relatively high (carbon dioxide)/(oxygen) selectivity. In some cases, membranes that allow more exhaled oxygen 28 than molecular anesthetic agent 34 to pass therethrough are advantageous for use in anesthetic circuit 10. Some membranes having this property are free of substances that are chemically reactive with exhaled carbon dioxide 30 (and possibly exhaled molecular anesthetic agent 34) that produce harmful by-products. Furthermore, the need to replace membranes when a chemically reactive material is used up can be avoided by the use of some membranes that allow exhaled oxygen 28 (optionally in substantial amounts) to pass therethrough.

It is advantageous to retain at least some (optionally a substantial amount) of relatively expensive exhaled molecular anesthetic agent 34 for re-inhalation by patient 20, while reducing (optionally substantially) the amount of exhaled carbon dioxide 30 in anesthetic circuit 10. Since exhaled carbon dioxide 30 is permitted to pass through membrane 38 and out of flow passage 12, this prevents the patient from re-inhaling excessive amounts of exhaled carbon dioxide 30, which could have detrimental health effects.

Exhaled molecular anesthetic agent 34 is a volatile anesthetic agent. Membrane 38 is at least partially (optionally, substantially) impervious (to the volatile anesthetic agent. Exhaled molecular anesthetic agent 34 may include a mixture of sevolfurane, isoflurane and/or desflurane. Membrane 38 may be at least partially (optionally, substantially) impervious to sevoflurane, isoflurane and/or desflurane.

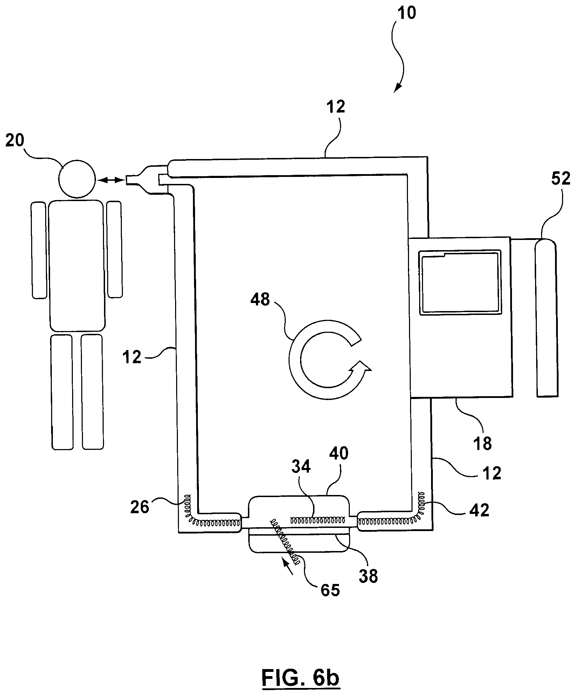

In some cases, as exemplified in Figure 6b, membrane 38 is configured such that secondary oxygen 65 located external to flow passage 12 passes through membrane 38 and into flow passage 12. Secondary oxygen 65 may be a natural component that is part of atmospheric air adjacent membrane 38, on a side of the membrane that is external to flow passage 12. Secondary oxygen 65 may also be introduced from an external source, such a compressed tank of air or substantially pure oxygen, for example.

In the context of the present application, the negligible amount of any anesthetic substances typically present in air are not considered to be anesthetic agents. External anesthetic agent 16 (see Figure 1) and exhaled molecular anesthetic agent 34, for the purposes of the present application, pertain to substances that are present in sufficient quantities to have (or at least appreciably contribute) to the anesthetic or desired protective effect on patient 20. Therefore, reference to an anesthetic agent refers to chemicals that are added to the naturally occurring constituents of air. In some embodiments, external anesthetic agent 16 comprises a mixture of different anesthetic agents and exhaled molecular anesthetic agent 34 comprises a mixture of different anesthetic agents.

By retaining some (or, optionally, a substantial amount) of exhaled molecular anesthetic agent 34 within flow passage 12, exhaled molecular anesthetic agent 34 can be re-circulated and re-inhaled by patient 20. Therefore, less costly external anesthetic agent 16 (see Figure 1) needs to be added to the flow passage 12 to keep the patient under the influence of the anesthetic. The amount of environmentally harmful exhaled molecular anesthetic agent 16 that is exhausted into the external atmosphere may be minimized.

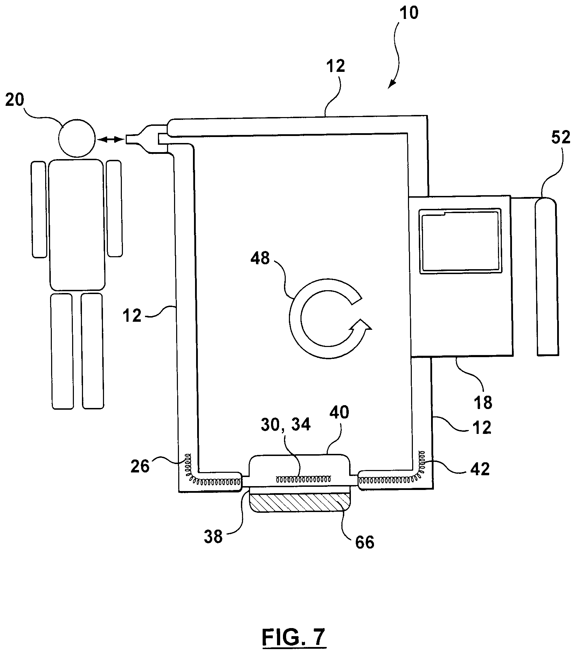

In the embodiment illustrated in Figure 7, anesthetic circuit 10 comprises a carbon dioxide absorbing material 66. Carbon dioxide absorbing material 66 may comprise at least one of: soda lime, alkanolime, alkanolamine, amino compounds, alkali salts of amino acids, glycine, DL-alanine, beta-alanine, serine, threonine, isoleucine, DL-valine, piperazine-2-carboxilic acid, proline, arginine, gamma-aminobutyric acid, ornithine, potassium glycinate, potassium threonate, taurine, creatine and histidine. Carbon dioxide absorbing material 66 absorbs exhaled carbon dioxide 30 from flow passage 12 and decreases the amount of exhaled carbon dioxide 30 that is re-introduced to patient 20. As exemplified in Figure 7, carbon dioxide absorbing material 66 is located on a side of membrane 38 that is external to flow passage 12. Membrane 38 separates carbon dioxide absorbing material 66 from exhaled molecular anesthetic agent 34 retained in flow passage 12 to impede the exhaled molecular anesthetic agent 34 from contacting the carbon dioxide absorbing material 66.

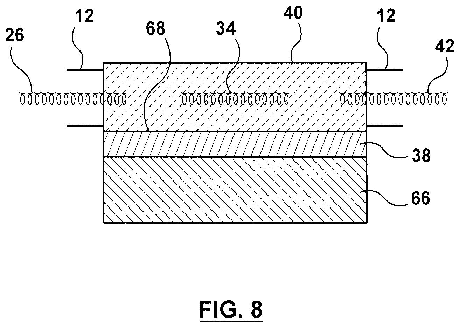

Figure 8 illustrates membrane 38 separating exhaled molecular anesthetic agent 34 in exhaled fluid mixture 26 from carbon dioxide absorbing material 66.

It is advantageous to have membrane 38 impede exhaled molecular anesthetic agent 34 from chemically interacting with carbon dioxide absorbing material 66. When exhaled molecular anesthetic agent 34 is sevoflurane and carbon dioxide absorbing material 66 is soda lime, for example, contact and interaction between exhaled molecular anesthetic agent 34 and carbon dioxide absorbing material 66 can create harmful by-products, such as compound A, which may have harmful effects on patient 20, if inhaled in sufficient quantities. Since membrane 38 selectively allows more exhaled carbon dioxide 30 to pass therethrough than exhaled molecular anesthetic agent 34, these harmful reactions are minimized, while still effectively absorbing and extracting the exhaled carbon dioxide 30 out of flow passage 12.

Membrane 38 is inert with respect to exhaled molecular anesthetic agent 34.

In some cases, membrane 38 is completely inert. In other words, membrane 38 is not chemically reactive with any other substances.

Membrane 38 may be free of any amino acids. In this case, no amino acids are impregnated into membrane 38 or deposited onto a surface of membrane 38.

When a membrane is impregnated with an amino acid or has amino acids deposited thereon, the amino acids react with the exhaled carbon dioxide 30. During this reaction, the amino acids may be consumed. Once the amino acids are consumed, the membrane 38 has to be replaced (or more amino acids added thereto). It is advantageous to have a membrane 38 that is inert and does not have to be replaced or replenished due to chemical degradation.

In some embodiments, exhaled fluid mixture 26 comprises a metabolic product (not shown) including acetaldehyde, acetone, ethane, ethylene, hydrogen, isoprene, methane, methylamine or pentane. In some cases, exhaled fluid mixture 26 comprises a metabolic product consisting of a mixture of two of more of the metabolic by products listed above. Membrane 38 may be pervious to the metabolic product to permeate the metabolic product through membrane 38, and out of flow passage 12. Optionally, membrane 38 has a metabolic product-to-exhaled molecular anesthetic agent 34 selectivity of greater than 1. In this case, exhaled fluid mixture 26 contacts membrane 38 to leave modified fluid mixture 42 in the flow passage having a lower amount of the metabolic product than exhaled fluid mixture 26. Membrane 38 may have a membrane product-to-exhaled molecular anesthetic selectivity of at least 2. Optionally, membrane 38 has a membrane product-to-exhaled molecular selectivity of at least 3, 4, 5, 10, 50, 100 or 250.

Example membranes for membrane 38 (shown in Figures 1-2, 4-5, and 6a-6b) will now be discussed in detail.

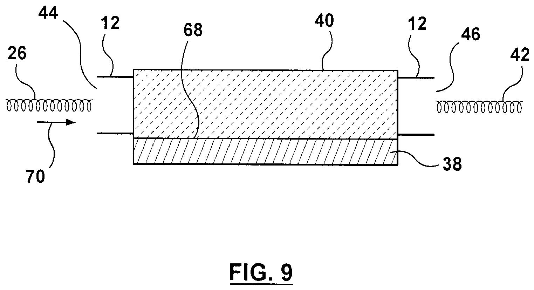

As exemplified in Figure 1, membrane 38 may be configured within anesthetic circuit 10 such that the exhaled fluid mixture 26 tangentially contacts an interior surface 68 of membrane 38. As exemplified in Figure 9, exhaled fluid mixture 26 has entry direction 70, as it enters housing inlet 44 of membrane housing 40. Although the flow direction of different portions of the exhaled fluid mixture 26 may be varied and the flow may be turbulent at this point, the exhaled fluid mixture 26 has an average direction indicated by entry direction 70, as defined by flow passage 12. In some cases, the longitudinal axis of membrane 38 is substantially parallel to entry direction 70 of exhaled fluid mixture 26, as shown in Figure 9.

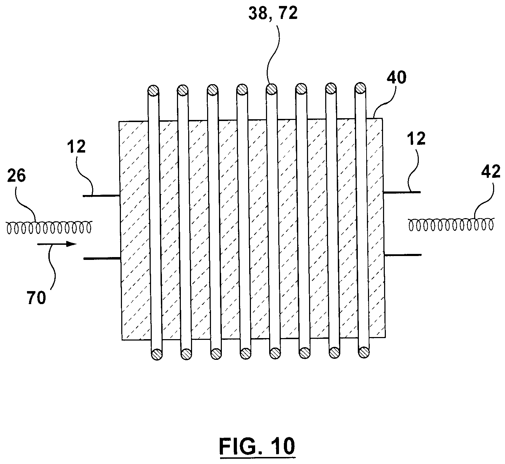

Figure 10 exemplifies the membrane 38 comprising hollow fibers 72. As shown in Figure 10, membrane 38 may be at least partially contained within membrane housing 40. As the exhaled fluid mixture 26 contacts the outer surface of a hollow fiber 72, at least exhaled oxygen 28 and exhaled carbon dioxide 30, selectively enter the hollow interior of at least one hollow fiber 72. The components (or portion thereof) that enter the interior of a hollow fiber 72 then pass through membrane 38 out of the flow passage 12 via at least one end of the hollow fiber 72. The portion of the exhaled fluid mixture 26 that remains exterior to the hollow fibers 72 exits housing 40 as modified fluid mixture 42. Typically, modified fluid mixture 42 has a lesser amount of any components (or portions thereof) lost through the ends of at least one hollow fiber 72. Optionally, the hollow fibers 72 are substantially orthogonal to the entry direction 70 of exhaled fluid mixture 26, as shown in Figure 10.

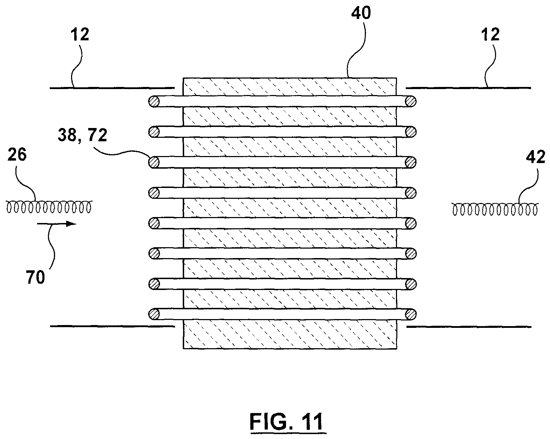

Alternatively, Figure 11 exemplifies hollow fibers 72 that are substantially parallel to the entry direction 70 of exhaled fluid mixture 26. As shown in Figure 11, membrane 38 may be at least partially contained within membrane housing 40. In this case, exhaled fluid mixture 26 enters the interior of a hollow fiber 72 via an end of a hollow fiber 72. The portion of the exhaled mixture that remains interior to the hollow fibers 72 exits the membrane 38 as modified fluid mixture 42. The components (or portion thereof) that selectively pass from the interior to the exterior of a hollow fiber 72 pass through membrane 38 and out of flow passage 12.

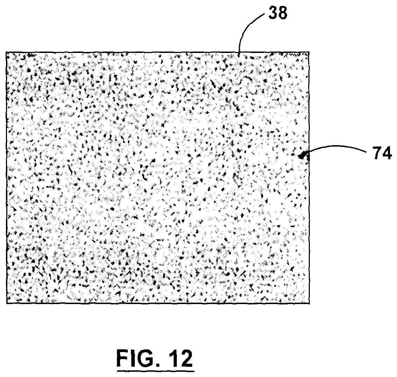

Membrane 38 comprises a dense membrane. In this case, membrane 38 is considered a dense membrane. In some cases, membrane 38 is entirely made of a dense membrane material. As will be understood by the skilled person, dense membranes comprise a solid material that is free of any pores or voids. A substance passes through a dense membrane by a process of solution and diffusion. The substance passes through membrane 38 by dissolving into membrane 38 and passing through to an opposite side thereof. An example dense membrane 38 is illustrated in Figure 12. Figure 12 exemplifies a membrane 38 that is a dense, non-porous membrane comprising a unitary solid layer 74 having a non-porous consistency therethrough. In some cases, membrane 38 is entirely made up of dense membrane material.

In some embodiments, membrane 38 is a dense membrane made of polymethylpentene. More specifically, unitary solid layer 74 (shown in Figure 12) may be made of polymethylpentene. In some cases, the membrane 38 comprises a dense membrane made of polymeric silicone. More specifically, membrane 38 may comprise polydimethyl siloxane. Dense membranes rely on solution and diffusion as principles of travel through the membrane and also for selectivity. As discussed in more detail below, polymethylpentene membranes were found to have a selectivity preference to carbon dioxide and oxygen, as opposed to molecular anesthetics. Since polymeric silicone, and more specifically, polydimethyl siloxane, are dense membranes like a polymethylpentene dense membrane, a similar selectivity is predicted.

An example polymethylpentene dense membrane may be used with the QUADROX-D^{™} oxygenator, for example. The QUADROX^{™} trademark is owned by MAQUET CARDIOPULMONARY AG^{™}. The QUADROX-D^{™} product is sold by MAQUET^{™}, which is part of the GETINGE AB^{™} group of companies. To the best of the Applicants knowledge, an oxygenator such as the QUADROX-D^{™} oxygenator has been used in on-pump cardiac surgeries. In some embodiments of the present invention, the QUADROX-D^{™} oxygenator is used as part of anesthetic circuit 10, as membrane housing 40 having membrane 38 therein (see Figure 1, for example).

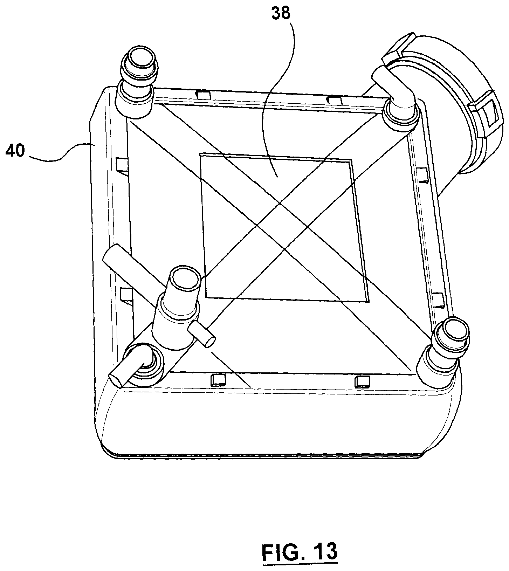

Figure 13 illustrates QUADROX-D^{™} having a membrane 38 disposed within a membrane housing 40. Membrane housing 40 for QUADROX-D^{™} is made of polycarbonate. QUADROX-D^{™} has a blood flow rate of approximately 0.5-7 l/min. The total priming volume is 250 ml, while the effective surface area for fluid exchange is approximately 1.8 m². The effective surface area for heat exchange is approximately 0.6 m². The oxygenation fibers are made of polymethylpentene. The heat exchange fibers and potting material are made of polyurethane. The protective caps are made of polyethylene.

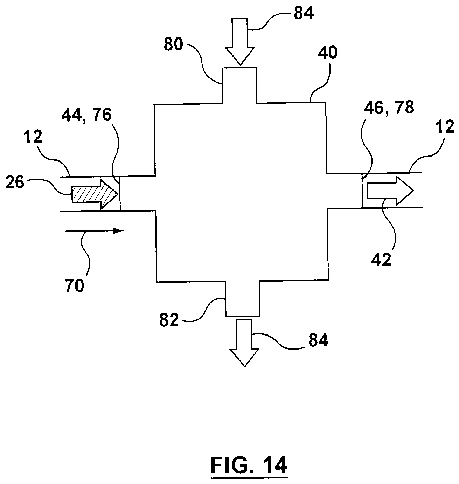

Figure 14 exemplifies an oxygenator similar in its basic operation to QUADROX-D^{™}, shown in Figure 13, comprises a membrane housing 40 having a blood inlet 76 and a blood outlet 78. For on-pump cardiac surgeries, blood enters membrane housing 40 via blood inlet 76, passes through the membrane in the housing (not shown), and exits the membrane housing 40 via blood outlet 78 in a modified form. Typically, the modified blood exits with a higher oxygen concentration and a lower carbon dioxide concentration. In an embodiment of the present invention, blood inlet 76 functions as housing inlet 44. As opposed to blood entering the housing inlet 44, exhaled fluid mixture 26 enters membrane housing 40 via housing inlet 44. In this embodiment, blood outlet 78 functions as housing outlet 46. As opposed to modified blood exiting the housing outlet 46, modified fluid mixture 42 exits the membrane housing 40 via housing outlet 46.

The oxygenator illustrated in Figure 14 also comprises a sweep inlet 80 and a sweep outlet 82. A sweep fluid 84 enters membrane housing 40 via sweep inlet 80 and exits the membrane housing 40 via sweep outlet 82. Optionally, the sweep inlet 80, housing outlet 46, sweep outlet 82, and housing inlet 44 are staggered on four separate, orthogonal walls such the flow of the sweep fluid 84 through membrane housing 40 is substantially orthogonal to the entry direction 70 of exhaled fluid mixture 26 into membrane housing 40. The sweep fluid 84 guides the exhaled fluid towards and into contact with the membrane (not shown) within membrane housing 40. Sweep fluid 84 may be, for example, air or substantially pure oxygen.

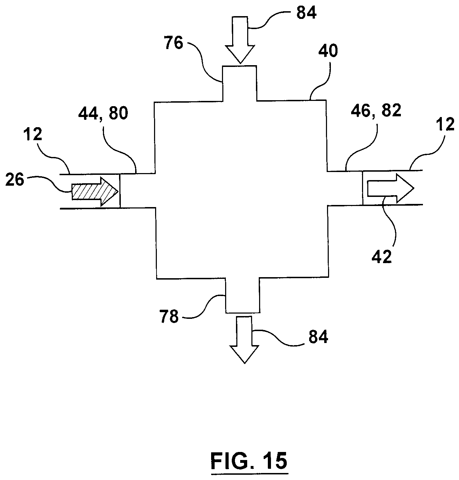

In another embodiment, as exemplified in Figure 15, the blood inlet 76 and the blood outlet 78 function as the inlet and outlet for the sweep fluid 84. In this embodiment, sweep inlet 80 and sweep outlet 82 function as housing inlet 44 and housing outlet 46, respectively. In the embodiment exemplified in Figure 15, the oxygenator similar in its basic operation to QUADROX-D^{™} has been rotated by 90° relative to the oxygenator exemplified in Figure 14. As exemplified in Figure 15, sweep inlet 80 and sweep outlet 82 engage flow passage 12.

Exhaled fluid mixture 26 contacts the membrane (not shown in Figure 15) within the membrane housing 40 such that exhaled fluid mixture 26 is converted to modified fluid mixture 42.

In some cases, the surface of membrane 38 within membrane housing 40 of an oxygenator, such as QUADROX-D^{™}, for example, is treated with SAFELINE^{™} treatment. In some cases, the surface of membrane 38 may be treated with BIOLINE^{™} coating. In some cases, the surface of membrane 38 is not treated with the SAFELINE^{™} or BIOLINE^{™} treatment.

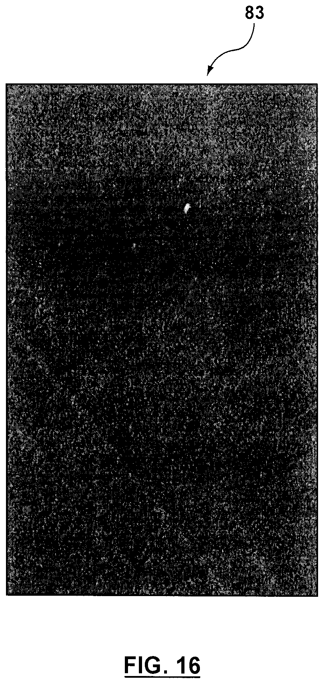

An example membrane 38 for use within an oxygenator, such as the QUADROX-D^{™} oxygenator, for example, is the OXYPLUS^{™} membrane. The OXYPLUS^{™} trademark is owned by MEMBRANA GMBH CORPORATION^{™}. OXYPLUS^{™} is a polyhalocarbon membrane. OXYPLUS^{™} is a hydrophobic polyolefin membrane. More specifically, OXYPLUS^{™} is a polymethylpentene membrane. OXYPLUS^{™} is an asymmetric membrane having a porous support layer made of polymethylpentene and a dense layer also made of polymethylpentene. It will be appreciated that such a membrane is referred to in the art as a dense membrane, due to the presence of the dense outer layer. In turn, membrane 38 may be a membrane made up of only polymethylpentene. The dense layer 83 may have a thickness of less than or equal to 1.5 micrometers, 1 micrometer or 0.5 micrometers. Due to the dense, non-porous nature of the dense layer 83, substances transfer through dense layer 83 by diffusion and solution, as is the conventional manner for a completely dense membrane or a dense layer. Figure 16 provides a plan view of dense layer 83 of the OXYPLUS^{™} membrane, magnified by 5000 times.

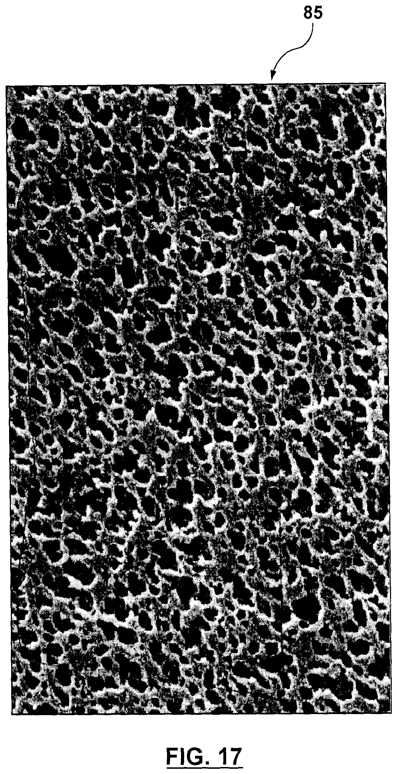

Figure 17 provides a plan view of porous support layer 85 of the OXYPLUS^{™} membrane, magnified by 5000 times.

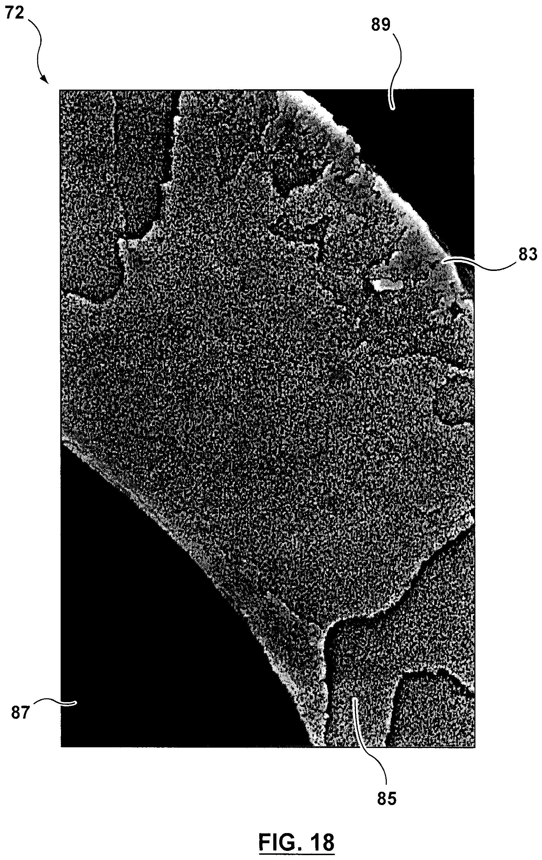

OXYPLUS^{™} is typically comprises hollow fibers 72, as illustrated in Figure 10 and 18. In this case, membrane 38 is an asymmetric membrane comprising hollow fibers 72 having at least one wall comprising a porous support layer and a dense layer. Figure 18 shows dense layer 83 along the a portion of the outer diameter of an OXYPLUS^{™} hollow fiber 72, and a portion of porous support layer 85 extending inwardly from dense layer 83. Figure 18 shows an OXYPLUS^{™} membrane magnified by 1000 times. Each hollow fiber 72 (a wall portion of which is shown in Figure 18) may have an outer diameter of approximately 380 micrometers (+ or - 10% or 20%) and an inner diameter of approximately 200 micrometers (+ or - 10% or 20%). In some embodiments, multiple hollow fibers 72 may be cross wound with one another, to maintain a fixed position relative to one another.

Continuing to refer to Figure 18, exhaled fluid mixture 26 may pass first through dense layer 83, then through porous support layer 85 and into the hollow fiber interior 87. In this manner, the OXYPLUS^{™} hollow fiber membrane operates in the manner discussed with respect to Figure 10. Alternatively, exhaled fluid mixture 26 may pass first through porous support layer 85, then though dense layer 83 and to the exterior 89 of the hollow fiber. In this manner, the OXYPLUS^{™} hollow fiber membrane operates in the manner discussed with respect to Figure 11.

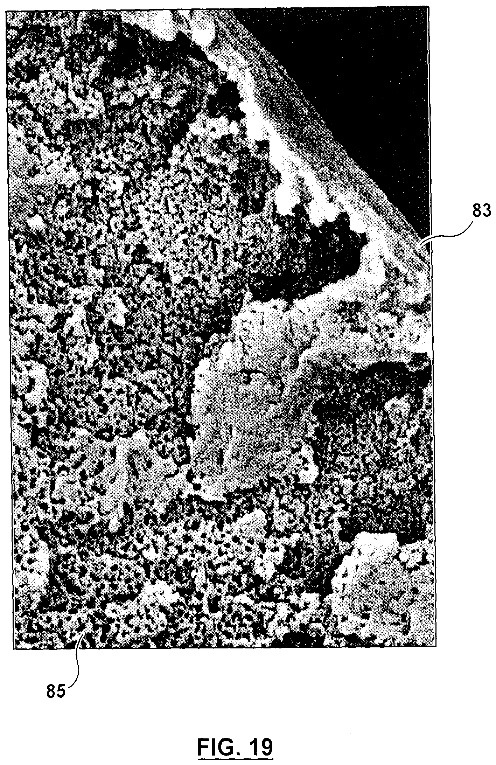

Figure 19 shows dense layer 83 and porous support layer 85 of the OXYPLUS^{™} membrane magnified by 5000 times.

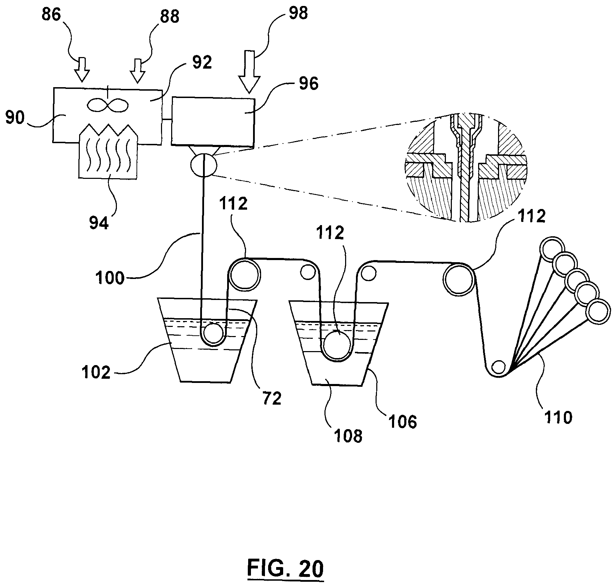

OXYPLUS^{™} is produced using the ACCUREL^{™} process. The ACCUREL^{™} process is a thermally induced phase separation process. Referring to Figure 20, polymer 86 is substantially homogenously melt-mixed with solvent 88 in mixer 90 to form mix 92, while being subjected to heat source 94. When producing OXYPLUS^{™}, polymer 86 may be polymethylpentene. The solvent 88 may comprise natural seed oils, such as soy and castor, for example. The mix 92 passes through heat extruder 96. A nitrogen fluid source 98 may be used to add nitrogen to mix 92 within heat extruder 96. Mix 92 then passes through a temperature controlled air gap 100, until it reaches spinning chamber 102. In spinning chamber 102, hollow fibers 72 of the OXYPLUS^{™} membrane are formed by spinning and cooling hollow fibers 72. During cooling, phase separation is initiated leading to the formation of a porous skeleton structure consisting of solid polymer. In spinning chamber 102, the pores are still filled with oil. The created hollow fibers 72 are then passed to extraction chamber 106. In extraction chamber 106, the oil residues are removed from the pores using hot alcohol 108. The hollow fibers 72 are then passed to a drying stage 110 at which the hollow fibers 72 are dried to form the OXPLUS^{™} membrane. Rotatable spools 112 may be used to guide the mix 92 and hollow fibers 72 through the process stages illustrated in Figure 20. During the production process for OXYPLUS^{™}, a dense layer 83 is created and disposed on porous support layer 85. Therefore, the OXYPLUS^{™} membrane comprises a porous support layer 85 surrounded by dense layer 83.

An alternative example membrane 38 is the ULTRAPHOBIC^{™} membrane produced by Membrana GmbH. Like OXYPLUS^{™}, ULTRAPHOBIC^{™} is a polyhalocarbon membrane. ULTRAPHOBIC^{™} is a hydrophobic polyolefin membrane.. More specifically, ULTRAPHOBIC^{™} is a polymethylpentene membrane having a polymethylpentene porous support layer 85 and a polymethylpentene dense layer 83.

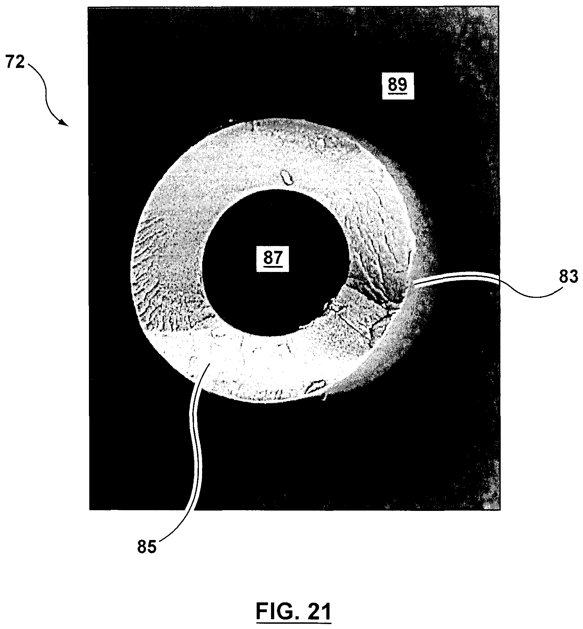

Figure 21 shows a microscopic view of hollow fiber 72 for the ULTRAPHOBIC^{™} membrane having a dense layer 83 and a porous support layer 85. Figure 21 shows hollow fiber interior 87 and hollow fiber exterior 89. ULTRAPHOBIC^{™} operates in the same manner as outlined above for the OXYPLUS^{™} membrane (with reference to Figures 16-19).

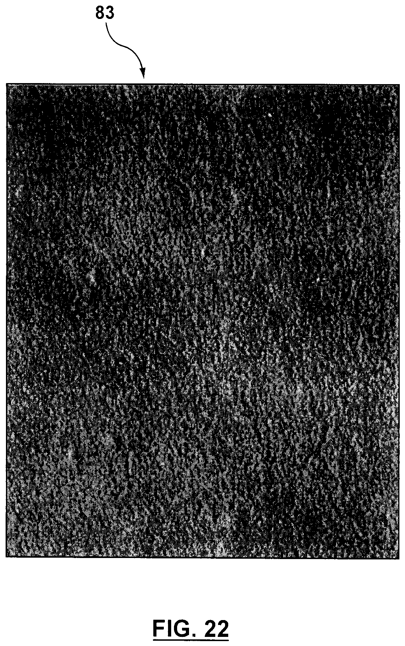

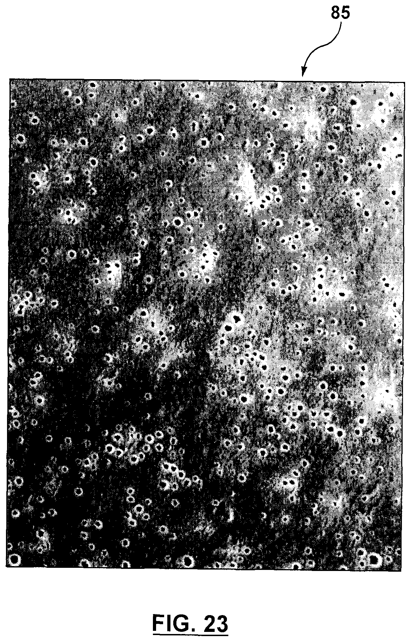

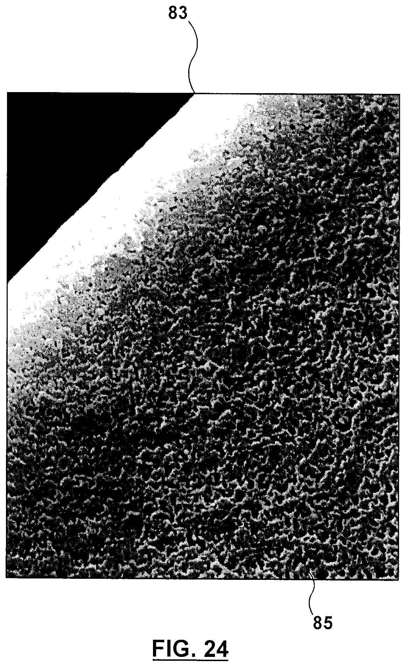

Figure 22 illustrates a microscopic view of dense layer 83 for the ULTRAPHOBIC^{™} membrane. Figure 23 illustrates a microscopic view of porous support layer 85 for the ULTRAPHOBIC^{™} membrane. Figure 24 shows an additional microscopic view of dense layer 83 and porous support layer 85 for the ULTRAPHOBIC^{™} membrane.

Membrane 38 may comprise a glassy polymer. More specifically, membrane 38 may comprise at least one of cellulose acetate, polymide and polysulfone. Glassy polymers are diffusivity selective, meaning that they permeate polar molecules with higher solubility in the membrane material (such as carbon dioxide and oxygen gases, for example) faster than nonpolar molecules with lower solubility in the membrane material (such as sevoflurane, desflurane and isoflurane vapors, for example).

More specifically, membrane 38 may comprise a high free volume glassy polymer. More specifically, membrane 38 may comprise at least one of PTMSP [i.e. poly(1-trimethlsilyl-1-propyne) and polymethylpentene. As described in more detail below, polymethylpentene membranes were found to have a selectivity preference to carbon dioxide and oxygen, as opposed to molecular anesthetics such as sevoflurane, isoflurane and isoflurane anesthetics. PTMSP, like polymethypentene, is a high volume glassy polymer and is expected to exhibit an affinity for oxygen and carbon dioxide selectivity, as opposed to molecular anesthetic selectivity. These membranes tend to preferentially permeate materials with relatively high condesability/solubility levels (such as oxygen and carbon dioxide gas, for example). Notably, the permeation of nonpolar hydrocarbons is much lower than that of polar organic species. High free volume glassy polymers have the advantage that the permeability/flux is higher than for normal glassy polymers.

Membrane 38 may comprise a polymeric size selective membrane. These membranes function based on a molecular sieving mechanism. They allow molecules smaller than the pore sizes of the membrane (ex. oxygen and carbon dioxide gas) to pass through the membrane, while larger molecules (ex. sevoflurane, desflurane and isoflurane vapors) are substantially retained by the membrane.

Membrane 38 may comprise a polymer composite or a polymer mixed matrix membrane. Composite membranes have more than one layer of substances with different permeability/selectivity. One layer may be, for example, a high free volume layer. Mixed matrix membranes have other phases/substances immobilized in a polymer matrix. Composite membranes can be tailed to have the characteristics of normal and high free volume glassy polymers, or a size selective membrane, as discussed above, or a combination thereof. Membrane 38 may comprise a composite POLARIS^{™} membrane. POLARIS^{™} is a product offered by Membrane Technology and Research, Inc.

Tests were conducted in which a QUADROX-D^{™} oxygenator was used in the set-up illustrated in Figure 2. In this set-up, membrane 38 (within the QUADROX-D^{™} oxygenator) was the OXYPLUS^{™} membrane. Specifically, an OXYPLUS^{™} 90/200 membrane having comprising hollow fibers 72 (see Figure 10) an outer diameter of 380 micrometers and a dense layer 83 having a thickness of less than 1 micrometer was used.

The results of one experiment are shown in Table 1. For this experiment, the oxygenator configuration illustrated in Figure 15 was used. Sweep fluid 84 was oxygen fluid. For test #1, exhaled molecular anesthetic agent 34 was tested separately as sevoflurane (SEVO) and isoflurane (ISO).

**Table 1: Results of Experiment #1 (Test #1)**

| | **flowrate** | **CO₂** | **O₂** | **SEVO** | **ISO** |
|---|---|---|---|---|---|
| | [l/min] | [%] | [%] | [%] | [%] |
| Sweep Fluid 84 | 2 | 0 | 100 | 0 | 0 |
| | | | | | |
| Exhaled Fluid Mixture 26 | 6 | 4.8 | 93 | 0.2 | 0.75 |
| Modified Fluid Mixture 42 | | 0.9 | 92 | 0.88 | 0.72 |
| Relative Change | | -3.9 | -1 | -0.04 | -0.03 |

Experiment #2 (tests #2-4) were also conducted in which a QUADROX-D^{™} oxygenator was used in an anesthetic circuit 10 having one (Figure 2), two, and three membrane(s) 38. When more than one membrane 38 is present, membranes 38 may be configured in series along flow passage 12, as shown in Figure 5, for example. In these set-ups, membrane 38 (within the QUADROX-D^{™} oxygenator) was the OXYPLUS^{™} membrane. Specifically, an OXYPLUS^{™} 90/200 membrane having hollow fibers 72 with an outer diameter of 380 micrometers and a dense layer 83 having a thickness of less than 1 micrometer was used.

The results of tests #2-4 are shown in Table 2. For this group of tests, the oxygenator configuration illustrated in Figure 14 was used. The sweep fluid 84 was air. Sweep fluid 84 had a flow rate of 30 l/min. Exhaled fluid mixture 26 had a flow rate of 7 l/min. For tests #2-4, exhaled molecular anesthetic agent 34 was sevoflurane (SEVO).

**Table 2: Results of Experiment #2 (Tests #2-4)**

| | **Test #2** | **Test #3** | **Test #4** |
|---|---|---|---|
| **Number of Membranes 38** | 1 | 2 | 3 |
| | | | |
| **Exhaled Fluid Mixture 26** | | | |
| CO₂ [%] | 4.8 | 4.8 | 4.8 |
| O₂ [%] | 93 | 93 | 93 |
| SEVO [%] | 1.6 | 1.6 | 1.6 |
| | | | |
| **Modified Fluid Mixture 42** | | | |
| CO₂ [%] | 1.2 | 0.3 | 0.1 |
| O₂ [%] | 86 | 63 | 48 |
| SEVO [%] | 2.1 | 2.9 | 3.4 |
| | | | |
| **Relative Change** | | | |
| CO₂ [%] | -3.6 | -4.5 | -4.7 |
| O₂ [%] | -7 | -30 | -45 |
| SEVO [%] | 0.5 | 1.3 | 1.8 |

Tests #5-8 were also conducted in which a QUADROX-D^{™} oxygenator was used in an anesthetic circuit 10 having one (Figure 2), two, three and four membrane(s) 38 (Figure 5). In these set-ups, membrane 38 (within the QUADROX-D^{™} oxygenator) was the OXYPLUS^{™} membrane. Specifically, an OXYPLUS^{™} 90/200 membrane having hollow fibers 72 with an outer diameter of 380 micrometers and a dense layer 83 having a thickness of less than 1 micrometer was used.

The results of tests #5-8 are shown in Table 3. For this group of tests, the oxygenator configuration illustrated in Figure 14 was used. The sweep fluid 84 was air. Sweep fluid 84 had a flow rate of 30 l/min. Exhaled fluid mixture 26 had a flow rate of 15 l/min.

**Table 3: Results of Experiment #3 (Test #5-8)**

| | **Test #5** | **Test #6** | **Test #7** | **Test #8** |
|---|---|---|---|---|
| **Number of Membranes 38** | 1 | 2 | 3 | 4 |
| **Exhaled Fluid Mixture 26** | | | | |
| CO₂ [%] | 4.8 | 4.8 | 4.8 | 4.8 |
| | | | | |
| **Modified Fluid Mixture 42** | | | | |
| CO₂ [%] | 2.8 | 1.4 | 0.7 | 0.6 |
| | | | | |
| **Relative Change** | | | | |
| CO₂ [%] | -2 | -3.4 | -4.1 | -4.2 |

A fourth experiment was conducted in which an oxygenator was used in the set-up illustrated in Figure 2. Membrane 38 was an OXYPLUS^{™} membrane.

For this experiment, membrane housing 40 resembled the configuration described above for Figure 10. As the exhaled fluid mixture 26 contacted the outer surface of a hollow fiber 72, the exhaled oxygen 28 and exhaled carbon dioxide 30, selectively entered the hollow interior of at least one hollow fiber 72. The components (or portion thereof) that entered the interior of a hollow fiber 72 then passed through membrane 38 out of the flow passage 12 via at least one end of the hollow fiber 72. The portion of the exhaled fluid mixture 26 that remained exterior to the hollow fibers 72 exited the membrane 38 as modified fluid mixture 42.

For these tests, sweep fluid 84 (see Figure 15) passed through the interior hollow fibers 72 to measure the amount of exhaled oxygen 28, exhaled carbon dioxide 30 and exhaled molecular anesthetic agent 34 in modified fluid mixture 42 (i.e. that passed into hollow fibers 72). The concentrations in the exhaled fluid mixture 26 were: 2% exhaled anesthetic 34 in oxygen and 4.8% exhaled carbon dioxide 30 in 94% exhaled oxygen 28. The flows were 0.8 L/min for sweep fluid 84 (see Figure 15) and 2.0 L/min for the exhaled fluid mixture 26. The concentrations in the exiting sweep fluid stream (i.e. modified fluid mixture 42) were measured using a patient monitor and a quadruple mass spectrometer. The patient monitor measurements are reflected in volume percentages, and the mass spectrometry measurements are reflected as Ion Currents in Amperes for the respective masses. By measuring the change between the original sweep fluid 84 that entered the inside of the hollow fibers from one end vs. the modified sweep fluid that that exited from the other end of the hollow fibers (after the exhaled fluid pass through the membrane's hollow fiber walls and into the hollow fibers) it was possible to discern the amount of exhaled molecular anesthetic, 02 and CO2 that passed through the membrane.

The results for experiment #4 are summarized in Table 4.

**Table 4: Results of Experiment #4**

| | | **Ion Current [A]** | | **Percentage** | |
|---|---|---|---|---|---|
| | **Mass** | **original** | **modified** | **original** | **modified** |
| **SEVO** | **Fluid Mixture** | | | | |
| | all membranes | 1.27E-09 | - | 2% | - |
| | **Sweep Fluid** | | | | |
| | Ultraphobic | 6.12E-11 | 6.12E-11 | 0% | 0% |
| | Oxyplus | 6.12E-11 | 1.09E-10 | 0% | 0.14% |

| **ISO** | **Fluid Mixture** | | | | |
|---|---|---|---|---|---|
| | all membranes | 9.49E-10 | - | 2% | - |
| | **Sweep Fluid** | | | | |
| | Ultraphobic | 6.72E-12 | 6.72E-12 | 0% | 0% |
| | Oxyplus | 6.72E-12 | 7.00E-11 | 0% | 0.16% |

| **DES** | **Fluid Mixture** | | | | |
|---|---|---|---|---|---|
| | all membranes | 1.06E-09 | - | 2% | - |
| | **Sweep Fluid** | | | | |
| | Ultraphobic | 5.44E-12 | 5.44E-12 | 0% | 0% |
| | Oxyplus | 5.44E-12 | 8.11E-11 | 0% | 0.15% |

| **CO2** | **Fluid Mixture** | | | | |
|---|---|---|---|---|---|
| | all membranes | 1.63E-09 | - | 4.80% | - |
| | **Sweep Fluid** | | | | |
| | Ultraphobic | 4.31E-11 | 2.43E-10 | 0% | 0.70% |
| | Oxyplus | 4.08E-11 | 7.73E-10 | 0% | 2.10% |

| **OXYGEN** | **Fluid Mixture** | | | | |
|---|---|---|---|---|---|
| | all membranes | 4.00E-09 | - | 94% | - |
| | **Sweep Fluid** | | | | |
| | Ultraphobic | 9.36E-10 | 1.07E-09 | 21% | 23% |
| | Oxyplus | 9.45E-10 | 1.85E-09 | 21% | 41% |

Also described herein is a method for anesthetic treatment of a patient. With reference to Figure 1, the method comprises introducing an external anesthetic agent 16 comprising a molecular anesthetic agent toward and into a patient via flow passage 12. External anesthetic agent 16 may be stored in anesthetic machine 18 or another external source. This anesthetic agent is delivered to patient 20, through flow passage 12. After the external anesthetic agent 16 is inhaled by patient 20, the external anesthetic agent 16 travels to the patient's lungs. The patient's lungs produce an exhaled fluid mixture 26, which is expelled from the airway of patient 20 as he/she exhales. Exhaled fluid mixture 26 is then directed away from and out of patient 20 into flow passage 12. Exhaled fluid mixture 26 from the patient 20 comprises exhaled oxygen 28, exhaled carbon dioxide 30 and exhaled molecular anesthetic agent 34. Exhaled fluid mixture 26 is advanced through flow passage 12 towards and into contact with membrane 38. Membrane 38 comprises a polymeric material and is in fluid communication with flow passage 12. More of each of exhaled carbon dioxide 30 and out of flow passage 12 than exhaled molecular anesthetic agent 34 is transferred through membrane 38 after exhaled fluid mixture 26 contacts membrane 38 to leave a modified fluid mixture 42 in flow passage 12. Modified fluid mixture 42 has a lower concentration of exhaled carbon dioxide 30 than exhaled fluid mixture 26. Oygen is transferred through membrane 38 after exhaled fluid mixture 26 contacts membrane 38 to leave modified fluid mixture 42 in flow passage 12. Membrane 38 has an exhaled oxygen-to-exhaled molecular anesthetic selectivity of greater than 1. Modified fluid mixture 42 is advanced through flow passage 12 toward patient 20 to provide at least modified fluid mixture 42 to patient 20.

Exhaled molecular anesthetic agent 34 is at least partially retained in flow passage 12 after exhaled fluid mixture 26 contacts the membrane 38. In some cases, substantially all (or substantial amounts) of the anesthetic agent 34 is retained in the flow passage 12 after exhaled fluid mixture 26 contacts the membrane 38.

Referring to Figure 6b, a secondary oxygen 65 located external to flow passage 12 may pass through membrane 38 and into flow passage 12. Secondary oxygen 65 comprises any oxygen that is external to flow passage 12, prior to operation and use of membrane 38. The external oxygen may be located on a side of membrane 38 that is external to flow passage 12. External oxygen may include, for example, oxygen naturally found in atmospheric air, or a source of oxygen located outside of flow passage 12 that is in fluid communication with membrane 38. In this scenario, the external oxygen serves to increase the total oxygen concentration in flow passage 12.

Referring to Figure 1, external fluid, which may be stored in external fluid source 52, is introduced into flow passage 12 through fluid inlet 50. In some cases, the external fluid that enters fluid inlet 50 may be enriched by oxygen from an external oxygen source 56 (see Figure 2).

Optionally, membrane 38 is pervious to exhaled oxygen 28 such that membrane 38 has an exhaled oxygen-to-exhaled molecular anesthetic agent selectivity of at least 2, 3, 4, 5, 10, 50, 100 or 250.

In the method, membrane 38 is pervious to exhaled carbon dioxide 30 such that membrane 38 has an exhaled carbon dioxide-to-exhaled molecular anesthetic agent selectivity of greater than 1. Optionally membrane 38 has a carbon dioxide-to-molecular anesthetic agent selectivity of at 2, 3, 4, 5, 10, 50, 100 or 250.

For implementation of the method of anesthetic treatment, membrane 38 is inert with respect to exhaled carbon dioxide 30.

In some cases, the membrane is fully operable, as outlined herein, at all humidity values ranging from 0% to 100%, including humidity values ranging from 0% to 100% within any fluid adjacent to an internal surface 68 of membrane 38 (see Figure 1).

While the present invention as herein shown and described in detail is fully capable of attaining the above-described objects of the invention, it is to be understood that it is the presently preferred embodiments of the present invention and thus, is representative of the subject matter which is broadly contemplated by the present invention, that the scope of the present invention as defined by the claims fully encompasses other embodiments which may become obvious to those skilled in the art, and that the scope of the claims should not be limited by the preferred embodiments set forth in the examples, but should be given the broadest interpretation consistent with the description as a whole. Moreover, it is not necessary for a device or method to address each and every problem sought to be solved by the present invention, for it is to be encompassed by the present claims.

## Claims

1. An anesthetic circuit (10) for treating a patient, comprising:
a flow passage (12);
a molecular anesthetic agent inlet (14) in fluid communication with the flow passage for introducing an external anesthetic agent comprising a volatile molecular anesthetic agent (16) into the flow passage;
an exit outlet (22) in fluid communication with the flow passage for providing at least the external anesthetic agent comprising a volatile molecular anesthetic agent to the patient;
an entry inlet (36) for receiving an exhaled fluid mixture (26) from the patient, the exhaled fluid mixture comprising an exhaled oxygen (28), an exhaled carbon dioxide (30) and an exhaled volatile molecular anesthetic agent (34), the flow passage being in fluid communication with the entry inlet for receiving the exhaled fluid mixture from the entry inlet;
a membrane (38) comprising at least one polymeric material, in fluid communication with the flow passage, located downstream from the entry inlet, and at least partially impervious to the exhaled volatile molecular anesthetic agent to at least partially retain the exhaled volatile molecular anesthetic agent in the flow passage after the exhaled fluid mixture contacts the membrane, wherein
the membrane comprises a dense membrane and has a non-porous consistency,
the membrane is pervious to the exhaled oxygen such that the membrane has an exhaled oxygen-to-exhaled volatile molecular anesthetic agent selectivity of greater than 1,
the membrane is pervious to the exhaled carbon dioxide such that the membrane has an exhaled carbon dioxide-to-exhaled volatile molecular anesthetic agent selectivity of greater than 1,
the membrane is capable, upon contact with the exhaled fluid mixture, of leaving a modified fluid mixture (42) in the flow passage having a lower amount of the exhaled carbon dioxide than the exhaled fluid mixture, and
the exit outlet is located downstream from the membrane and provides at least the modified fluid mixture to the patient (20); and
the membrane is inert with respect to the exhaled volatile molecular anesthetic agent and inert with respect to the exhaled carbon dioxide; and
a fluid inlet (50) for introducing an external fluid into the flow passage to be added to the modified fluid mixture provided to the patient.

2. The anesthetic circuit of claim 1 wherein the membrane (38) has an exhaled oxygen-to-exhaled volatile molecular anesthetic agent selectivity of at least 2.

3. The anesthetic circuit (10) of any one of claims 1 to 2 wherein the membrane (38) has an exhaled carbon dioxide-to-exhaled volatile molecular anesthetic agent selectivity of at least 2.

4. The anesthetic circuit (10) of any one of claims 1 to 3 wherein the membrane (38) is entirely made up of polymeric material.

5. The anesthetic circuit (10) of any one of claims 1 to 4 further comprising an external oxygen source (56) for enriching the external fluid with external oxygen.

6. The anesthetic circuit (10) of any one of claims 1 to 5 further comprising a turbulence-inducing component (64) in the flow passage (12) to create a turbulent flow of the exhaled fluid mixture at the membrane (38) to increase contact between the exhaled fluid mixture and the membrane.

7. The anesthetic circuit (10) of any one of claims 1 to 6 wherein the exhaled volatile molecular anesthetic agent:
comprises a polyhalogenated ether,
includes at least one of sevoflurane, isoflurane or desflurane, or
has a molecular weight of greater than 168 g/mol.

8. The anesthetic circuit (10) of any one of claims 1 to 7 wherein a carbon dioxide absorbing material (66) is located on a side of the membrane (38) that is external to the flow passage (12), wherein the membrane is capable of separating the carbon dioxide absorbing material from the exhaled volatile molecular anesthetic agent retained in the flow passage so as to impede the exhaled volatile molecular anesthetic agent from contacting the carbon dioxide absorbing material.

9. The anesthetic circuit (10) of claim 8 wherein the carbon dioxide absorbing material comprises at least one of: soda lime, alkanolime, alkanolamine, amino compounds, alkali salts of amino acids, glycine, DL-alanine, beta-alanine, serine, threonine, isoleucine, DL-valine, piperazine-2-carboxilic acid, proline, arginine, gamma-aminobutyric acid, ornithine, potassium glycinate, potassium threonate, taurine, creatine and histidine.

10. The anesthetic circuit (10) of any one of claims 1 to 9 wherein
the exhaled fluid mixture comprises a metabolic product including acetaldehyde, acetone, ethane, ethylene, hydrogen, isoprene, methane, methylamine or pentane;
the membrane (38) is pervious to the metabolic product; and
the membrane is capable, upon contact with an exhaled fluid mixture of leaving a modified fluid mixture (42) in the flow passage (12) having a lower amount of the metabolic product than the exhaled fluid mixture.

11. The anesthetic circuit (10) of any one of claims 1 to 10, wherein the membrane (38) is a:
polyhalocarbon membrane,
polymethylpentene membrane,
polysiloxane membrane, or
polydimethyl siloxane membrane.

12. The anesthetic circuit (10) of any one of claims 1 to 10 wherein the membrane (38):
is an asymmetric membrane comprising hollow fibers having at least one wall comprising a porous support layer and a dense layer, or
comprises a glassy polymer, a polymeric size selective membrane or a composite polymer membrane.

13. The anesthetic circuit (10) of any one of claims 1 to 12 wherein the membrane (38) is free of any amino acids.

## Patentansprüche

1. Anästhetikumkreislauf (10) zum Behandeln eines Patienten, umfassend:
einen Strömungskanal (12);
einen Einlass für ein molekulares anästhetisches Mittel (14) in fluider Kommunikation mit dem Strömungskanal zum Einführen eines externen anästhetischen Mittels, umfassend ein flüchtiges molekulares anästhetisches Mittel (16) in den Strömungskanal;
einen Ausgangsauslass (22) in fluider Kommunikation mit dem Strömungskanal zum Bereitstellen mindestens des externen anästhetischen Mittels, umfassend ein flüchtiges molekulares anästhetisches Mittel für den Patienten;
einen Eintrittseinlass (36) zum Aufnehmen eines ausgeatmeten Fluidgemisches (26) von dem Patienten, wobei das ausgeatmete Fluidgemisch einen ausgeatmeten Sauerstoff (28), ein ausgeatmetes Kohlendioxid (30) und ein ausgeatmetes flüchtiges molekulares anästhetisches Mittel (34) umfasst, wobei der Strömungskanal in fluider Kommunikation mit dem Eintrittseinlass zum Aufnehmen des ausgeatmeten Fluidgemisches von dem Eintrittseinlass ist;
eine Membran (38), umfassend mindestens ein polymeres Material, in fluider Kommunikation mit dem Strömungskanal, angeordnet stromabwärts von dem Eintrittseinlass, und mindestens teilweise undurchlässig für das ausgeatmete flüchtige molekulare anästhetische Mittel, um mindestens teilweise das ausgeatmete flüchtige molekulare anästhetische Mittel in dem Strömungskanal zurückzuhalten, nachdem das ausgeatmete Fluidgemisch die Membran kontaktiert, wobei
die Membran eine dichte Membran umfasst und eine nicht-poröse Konsistenz aufweist,
die Membran für den ausgeatmeten Sauerstoff durchlässig ist, sodass die Membran eine Selektivität von ausgeatmetem Sauerstoff-zu-ausgeatmetem flüchtigem molekularem anästhetischem Mittel größer als 1 aufweist,
die Membran für das ausgeatmete Kohlendioxid durchlässig ist, sodass die Membran eine Selektivität von ausgeatmetem Kohlendioxid-zu-ausgeatmetem flüchtigem molekularem anästhetischem Mittel größer als 1 aufweist,
die Membran befähigt ist, bei Kontakt mit dem ausgeatmeten Fluidgemisch ein modifiziertes Fluidgemisch (42) in dem Strömungskanal mit einer geringeren Menge an dem ausgeatmeten Kohlendioxid als das ausgeatmete Fluidgemisch zu hinterlassen, und
der Ausgangsauslass sich stromabwärts von der Membran befindet und mindestens das modifizierte Fluidgemisch für den Patienten (20) bereitstellt; und
die Membran im Hinblick auf das ausgeatmete flüchtige molekulare anästhetische Mittel inert ist und im Hinblick auf das ausgeatmete Kohlendioxid inert ist; und
einen Fluideinlass (50) zum Einführen eines externen Fluids in den Strömungskanal, das zu dem für den Patienten bereitgestellten modifizierten Fluidgemisch zuzugeben ist.

2. Anästhetikumkreislauf nach Anspruch 1, wobei die Membran (38) eine Selektivität von ausgeatmetem Sauerstoff-zu-ausgeatmetem flüchtigem molekularem anästhetischem Mittel von mindestens 2 aufweist.

3. Anästhetikumkreislauf (10) nach einem der Ansprüche 1 bis 2, wobei die Membran (38) eine Selektivität von ausgeatmetem Kohlendioxid-zu-ausgeatmetem flüchtigem molekularem anästhetischem Mittel von mindestens 2 aufweist.

4. Anästhetikumkreislauf (10) nach einem der Ansprüche 1 bis 3, wobei die Membran (38) vollständig aus polymerem Material hergestellt ist.

5. Anästhetikumkreislauf (10) nach einem der Ansprüche 1 bis 4, weiterhin umfassend eine externe Sauerstoffquelle (56) zum Anreichern des externen Fluids mit externem Sauerstoff.

6. Anästhetikumkreislauf (10) nach einem der Ansprüche 1 bis 5, weiterhin umfassend eine Turbulenz-induzierende Komponente (64) in dem Strömungskanal (12) zum Erzeugen eines Turbulenzstroms des ausgeatmeten Fluidgemisches an der Membran (38) zum Erhöhen des Kontakts zwischen dem ausgeatmeten Fluidgemisch und der Membran.

7. Anästhetikumkreislauf (10) nach einem der Ansprüche 1 bis 6, wobei das ausgeatmete flüchtige molekulare anästhetische Mittel:
einen polyhalogenierten Ether umfasst,
mindestens eines von Sevofluran, Isofluran oder Desfluran enthält oder ein Molekulargewicht von größer als 168 g/Mol aufweist.

8. Anästhetikumkreislauf (10) nach einem der Ansprüche 1 bis 7, wobei ein Kohlendioxid absorbierendes Material (66) sich auf einer Seite der Membran (38) befindet, die extern zu dem Strömungskanal (12) ist, wobei die Membran befähigt ist, das Kohlendioxid absorbierende Material von dem ausgeatmeten flüchtigen molekularen anästhetischen Mittel, das in dem Strömungskanal zurückgehalten wird, zu trennen, so dass dem ausgeatmeten flüchtigen molekularen anästhetischen Mittel das Kontaktieren des Kohlendioxid absorbierenden Materials erschwert wird.

9. Anästhetikumkreislauf (10) nach Anspruch 8, wobei das Kohlendioxid absorbierende Material mindestens eines umfasst von: Natronkalk, Alkanolime, Alkanolamin, Aminoverbindungen, Alkalisalze von Aminosäuren, Glycin, DL-Alanin, beta-Alanin, Serin, Threonin, Isoleucin, DL-Valin, Piperazin-2-carbonsäure, Prolin, Arginin, gamma-Aminobuttersäure, Ornithin, Kaliumglycinat, Kaliumthreonat, Taurin, Kreatin und Histidin.

10. Anästhetikumkreislauf (10) nach einem der Ansprüche 1 bis 9, wobei
das ausgeatmete Fluidgemisch ein metabolisches Produkt umfasst, enthaltend Acetaldehyd, Aceton, Ethan, Ethylen, Wasserstoff, Isopren, Methan, Methylamin oder Pentan;
die Membran (38) für das metabolische Produkt durchlässig ist; und
die Membran bei Kontakt mit einem ausgeatmeten Fluidgemisch ein modifiziertes Fluidgemisch (42) in dem Strömungskanal (12) mit einer geringeren Menge an dem metabolischen Produkt als das ausgeatmete Fluidgemisch hinterlassen kann.

11. Anästhetikumkreislauf (10) nach einem der Ansprüche 1 bis 10, wobei die Membran (38) eine:
Polyhalogenkohlenstoff-Membran,
Polymethylpenten-Membran,
Polysiloxan-Membran oder
Polydimethylsiloxan-Membran ist.

12. Anästhetikumkreislauf (10) nach einem der Ansprüche 1 bis 10, wobei die Membran (38):
eine asymmetrische Membran ist, umfassend Hohlfasern mit mindestens einer Wand, umfassend eine poröse Trägerschicht und eine dichte Schicht, oder
ein Kunstglas, eine größenselektive Polymer-Membran oder eine Verbundwerkstoff-Polymer-Membran umfasst.

13. Anästhetikumkreislauf (10) nach einem der Ansprüche 1 bis 12, wobei die Membran (38) frei von jeglichen Aminosäuren ist.

## Revendications

1. Circuit (10) anesthésique pour traiter un patient, comprenant:
un passage d'écoulement (12);
une entrée (14) d'agent anesthésique moléculaire en communication fluidique avec le passage d'écoulement pour introduire un agent anesthésique externe comprenant un agent (16) anesthésique moléculaire volatil dans le passage d'écoulement;
une sortie (22) en communication fluidique avec le passage d'écoulement pour fournir au moins l'agent anesthésique externe comprenant un agent anesthésique moléculaire volatil au patient;
une entrée (36) pour recevoir un mélange (26) de fluides exhalés par le patient, le mélange de fluides exhalés comprenant de l'oxygène (28) exhalé, du dioxyde de carbone (30) exhalé et un agent (34) anesthésique moléculaire volatile exhalé, le passage d'écoulement étant en communication fluidique avec l'entrée pour recevoir le mélange de fluides exhalés depuis l'entrée;
une membrane (38) comprenant au moins un matériau polymère, en communication fluidique avec le passage d'écoulement, située en aval de l'entrée et au moins partiellement imperméable à l'agent anesthésique moléculaire volatile exhalé pour retenir au moins partiellement l'agent anesthésique moléculaire volatile exhalé dans le passage d'écoulement après que le mélange de fluides exhalés entre en contact avec la membrane, dans lequel
la membrane comprend une membrane dense et présente une consistance non poreuse ;
la membrane est perméable à l'oxygène exhalé de sorte que la membrane a une sélectivité pour l'oxygène exhalé comparée à l'agent anesthésique moléculaire volatile exhalé supérieure à 1, la membrane est perméable au dioxyde de carbone exhalé de sorte que la membrane a une sélectivité pour le dioxyde de carbone exhalé comparée à l'agent anesthésique moléculaire volatile exhalé supérieure à 1,
la membrane est capable, en contact avec mélange de fluides exhalés, de laisser un mélange (42) modifié de fluides dans le passage d'écoulement ayant une quantité inférieure de dioxyde de carbone exhalé que de mélange de fluides exhalés et
la sortie est située en aval de la membrane et fournit au moins le mélange modifié de fluides au patient (20); et
la membrane est inerte en ce qui concerne l'agent anesthésique moléculaire volatile exhalé et inerte en ce qui concerne le dioxyde de carbone exhalé, et
une entrée (50) de fluide pour introduire un fluide externe dans le passage d'écoulement à ajouter au mélange modifié de fluides fourni au patient.

2. Circuit anesthésique selon la revendication 1 dans lequel la membrane (38) a une sélectivité pour l'oxygène exhalé comparée à l'agent anesthésique moléculaire volatile exhalé d'au moins 2.

3. Circuit (10) anesthésique selon la revendication 1 à 2, dans lequel la membrane (38) a une sélectivité pour le dioxyde de carbone exhalé comparée à l'agent anesthésique moléculaire volatile exhalé d'au moins 2.

4. Circuit (10) anesthésique selon l'une quelconque des revendications 1 à 3, dans lequel la membrane (38) est entièrement constituée de matériau polymère.

5. Circuit (10) anesthésique selon l'une quelconque des revendications 1 à 4, comprenant en outre une source (56) d'oxygène externe pour enrichir le fluide externe avec de l'oxygène externe.

6. Circuit (10) anesthésique selon l'une quelconque des revendications 1 à 5, comprenant en outre un composant (64) inducteur de turbulence dans le passage (12) d'écoulement pour créer un écoulement turbulent du mélange de fluides exhalés au niveau de la membrane (38) pour augmenter le contact entre le mélange de fluides exhalés et la membrane.

7. Circuit (10) anesthésique selon l'une quelconque des revendications 1 à 6, dans lequel l'agent anesthésique moléculaire volatil exhalé:
comprend un éther polyhalogéné,
inclut au moins l'un parmi le sévoflurane, l'isoflurane ou le desflurane ou présente un poids moléculaire supérieur à 168 g/mole.

8. Circuit (10) anesthésique selon l'une quelconque des revendications 1 à 7, dans lequel un matériau (66) absorbant le dioxyde de carbone est situé sur un côté de la membrane (38) qui est externe au passage (12) d'écoulement, dans lequel la membrane est capable de séparer le matériau absorbant le dioxyde de carbone de l'agent anesthésique moléculaire volatile exhalé retenu dans le passage d'écoulement pour empêcher l'agent anesthésique moléculaire volatile exhalé de se mettre en contact avec le matériau absorbant le dioxyde de carbone.

9. Circuit (10) anesthésique selon la revendication 8, dans lequel le matériau absorbant le dioxyde de carbone comprend au moins l'un parmi: le silicate de soude, l'alcanolime, l'alcanolamine, des composés amino, des sels alcalins d'acides aminés, la glycine, la DL-alanine, la bêta-alanine, la sérine, la thréonine, l'isoleucine, la DL-valine, l'acide pipérazine-2-carboxylique, la proline, l'arginine, l'acide gamma-aminobutyrique, l'ornithine, le glycinate de potassium, le thréonate de potassium, la taurine, la créatine et l'histidine.

10. Circuit (10) anesthésique selon l'une quelconque des revendications 1 à 9, dans lequel le mélange de fluides exhalé comprend un produit métabolique incluant l'acétaldéhyde, l'acétone, l'éthane, l'éthylène, l'hydrogène, l'isoprène, le méthane, la méthylamine ou le pentane;
la membrane (38) est perméable au produit métabolique; et
la membrane est capable, en contact avec mélange de fluide exhalé, de laisser un mélange (42) modifié de fluides dans le passage (12) d'écoulement ayant une quantité inférieure de dioxyde de carbone exhalé que le mélange de fluides exhalés.

11. Circuit (10) anesthésique selon l'une quelconque des revendications 1 à 10, dans lequel la membrane (38) est une:
membrane polyhalocarbonée,
membrane de polyméthylpentène,
membrane de polysiloxane, ou
une membrane de polydiméthylsiloxane.

12. Circuit (10) anesthésique selon l'une quelconque des revendications 1 à 10, dans lequel la membrane (38):
est une membrane asymétrique comprenant des fibres creuses ayant au moins une paroi comprenant une couche de support poreuse et une couche dense, ou
comprend un polymère vitreux, une membrane polymère sélective selon la taille ou une membrane polymère composite.

13. Circuit (10) anesthésique selon l'une quelconque des revendications 1 à 12, dans lequel la membrane (38) ne contient aucun acide aminé.
